# EUROPEAN PATENT APPLICATION

(11) **EP 3 339 858 A1**
(43) Date of publication of application: **27.06.2018**
(21) Application number: 16836681.3
(22) Date of filing: 19.08.2016
(51) Int. Cl.: G01N 33/48, G01N 33/64, G01N 33/70, G01N 33/74

(54) **CORONARY HEART DISEASE BIOMARKER AND APPLICATION THEREOF**

(30) Priority: 20.08.2015 WO PCT/CN2015/087664
(71) Applicant: BGI Shenzhen, Shenzhen, Guangdong 518083 (CN)
(72) Inventor: LI, Fengyu, Shenzhen Guangdong 518083 (CN); LI, Guanglei, Shenzhen Guangdong 518083 (CN); JIE, Zhuye, Shenzhen Guangdong 518083 (CN); ZHONG, Shilong, Guangzhou Guangdong 510080 (CN)
(74) Representative: dompatent von Kreisler Selting Werner - Partnerschaft von Patent- und Rechtsanwälten mbB
(86) International application number: PCT/CN2016/096090
(87) International publication number: WO 2017/028817

(57) **Abstract**

The present invention provides a coronary heart disease (CHD) biomarker and application thereof. Specifically, a biomarker set is provided. The set comprises multiple kinds of biomarkers and may be used for the CHD risk assessment of an object to be tested or for the CD diagnosis of the object to be tested. The present invention also provides a kit containing the biomarker set and the application of the biomarker set in CHD risk assessment and CHD diagnosis.

## Description

### Cross reference to related applications

This patent application claims the priority of PCT application (application number PCT/CN2015/087664) filed on August 20, 2015, which is incorporated herein by reference.

### Technical Field

The present invention relates to the field of biomedicine, specifically to cardiovascular disease biomarkers and the applications thereof, and more specifically to coronary heart disease (CHD) biomarkers and the applications thereof.

### Technical Background

Coronary heart disease (CHD) is the greatest health influence factor in modern society and causes more deaths each year than the sum of any other types of cancers. It was confirmed that the majority of the deaths of patients with cardiovascular diseases are related to the lack of awareness of their own health status and the lack of in-time treatment as demonstrated by a five-year follow-up study by MaGiCAD cohort. (Graninger, D.J. &Mosedale, D.E. Metabolomics in coronary heart disease.Heart.Metab.55, 8-12 (2012), incorporated herein by reference). The challenge for early diagnosis and prevention of CHD lies in the lackness of reliable non-invasive biomarkers. Currently, the "gold standard" for the diagnosis of CHD is still coronary angiography, which is invasive and has multiple risks of fatal side effects. This limits the screening and early risk prediction of CHD in large groups.

Many studies have pointed out that fatty acids play an important role in the metabolism of heart. They are main substrates of heart's ATP generation through the oxidative phosphorylation of mitochondria under normal physiological conditions, accounting for 60-90% of the heart's ATP synthesis. Cardiovascular disease (CVD), such as CHD and heart failure, undergoes a process of "metabolic changes", which is a result of both internal and external interference. Previously, the increase of low-density lipoprotein cholesterol (LDL-C) was considered as one of the major risk factors for CHD. In fact, the core defect in cardiovascular disease is lipid metabolism (Fernandez, C. et al. Plasma lipid composition and risk of developing cardiovascular disease. PLoS ONE 8, e71846 (2013), incorporated herein by reference), which makes metabonomics a particularly promising way to study such diseases.

Metabolomics is an innovative and high-throughput bioanalysis method designed to identify and quantify small molecules (molecular weight less than 1500 Daltons) that exist in any biological system or any specific physiological status. During the past decade, two major analytical techniques, nuclear magnetic resonance (NMR) and mass spectrometry (MS), have been shown an exponential growth in the measurement of endogenous compounds. MS-based techniques have made a considerable progress and have been used more frequently compared with NMR since 2005 because of the following advantages:: higher sensitivity, higher metabolome coverage, higher capabilities of metabolite identification and discrimination, and the modularization of component-classification-specific analysis (Griffiths, W.J. et al. Targeted metabolomics for biomarker discovery. Angew.Chem. Int. Ed. 49, 5426-5445 (2010), incorporated herein by reference). MS is mainly used in conjunction with chromatographic methods such as gas chromatography mass spectrometry (GC-MS) and liquid chromatography mass spectrometry (LC-MS).

At present, there are many deficiencies in the research on metabolomics for coronary heart disease, and there is an urgent need in the art for a further research on coronary heart disease metabolomics, especially coronary heart disease biomarkers.

### Summary of the Invention

The purpose of the present invention is to provide coronary heart disease (CHD) biomarkers and the applications thereof.

In the first aspect, the present invention provides a set of biomarkers comprising one or more biomarker(s) selected from the list consisting of:

| **Number** | **Name** | **Molecular Formula** | **m/z** | **retention time RT (s)** |
|---|---|---|---|---|
| Biomarker b1 | 1-Naphthol | C₁₀H₈O | 145.0649552 | 531.467 |
| Biomarker b2 | 2-Naphthol | C₁₀H₈O | 144.0571958 | 531.9175 |
| Biomarker b3 | L-Carnitine | C₇H₁₅NO₃ | 162.1122492 | 114.494 |
| Biomarker b4 | Methylitaconate | C₆H₈O₄ | 167.0315121 | 104.76 |
| Biomarker b5 | N-Acetyl-D-glucosamine 6-phosphate | C₈H₁₆NO₉P | 324.0458651 | 541.3545 |
| Biomarker b6 | 2,3-Dimethylmaleate | C₆H₈O₄ | 145.0496085 | 1156.85 |
| Biomarker b7 | Phenylpyruvate | C₉H₈O₃ | 165.0548439 | 183.8775 |
| Biomarker b8 | 20,26-Dihydroxyecdysone | C₂₇H₄₄O₈ | 496.3058791 | 744.104 |
| Biomarker b9 | Cortol | C₂₁H₃₆O₅ | 386.2897576 | 662.962 |
| Biomarker b10 | 10-Hydroxydihydrosanguinarine | C₂₀H₁₅NO₅ | 372.0845309 | 684.837 |
| Biomarker b11 | Progesterone | C₂₁H₃₀O₂ | 337.215992 | 774.709 |
| Biomarker b12 | (9Z,11E)-(13S)-13-Hydroperoxyoctadeca-9,11-dienoic acid | C₁₈H₃₂O₄ | 330.2635353 | 653.076 |
| Biomarker b13 | Phytosphingosine | C₁₈H₃₉NO₃ | 318.2999289 | 656.764 |
| Biomarker b14 | p-Coumaroylagmatine | C₁₄H₂₀N₄O₂ | 315.1211881 | 145.31 |
| Biomarker b15 | N6-(L-1,3-Dicarboxypropyl)-L-lysine | C₁₁H₂₀N₂O₆ | 315.096019 | 142.1965 |
| Biomarker b16 | Dihydrokaempferol | C₁₅H₁₂O₆ | 311.0511119 | 611.26 |
| Biomarker b17 | Indoleglycerol phosphate | C₁₁H₁₄NO₆P | 310.0474631 | 611.2495 |
| Biomarker b18 | Medicarpin | C₁₆H₁₄O₄ | 309.0542143 | 611.434 |
| Biomarker b19 | 2-Carboxy-2-hydroxy-8-carboxychromene | C₁₁H₈O₆ | 274.9929078 | 91.3841 |
| Biomarker b20 | Hexadecanoic acid | C₁₆H₃₂O₂ | 274.2736949 | 656.679 |
| Biomarker b21 | Xanthoxin | C₁₅H₂₂O₃ | 268.1909849 | 571.68 |
| Biomarker b22 | 5-(3'-Carboxy-3'-oxopropyl)-4,6-dihydroxypicolinate | C₁₀H₉NO₇ | 255.0378919 | 117.92 |
| Biomarker b23 | N-Acetyl-L-2-amino-6-oxopimelate | C₉H₁₃NO₆ | 249.1082592 | 117.643 |
| Biomarker b24 | 4-(L-Alanin-3-yl)-2-hydroxy-cis, cis-muconate 6-semialdehyde | C₉H₁₁NO₆ | 247.0927331 | 146.379 |
| Biomarker b25 | 4-[(Hydroxymethyl)nitrosoamino] -1-(3-pyridinyl)- 1-butanone | C₁₀H₁₃N₃O₃ | 223.0935157 | 644.408 |
| Biomarker b26 | Ecgonine | C₉H₁₅NO₃ | 203.1391048 | 119.338 |
| Biomarker b27 | Creatine | C₄H₉N₃O₂ | 170.0328456 | 202.182 |
| Biomarker b28 | 7-Methylxanthine | C₆H₆N₄O₂ | 167.0564801 | 502.8765 |
| Biomarker b29 | Cyromazine | C₆H₁₀N₆ | 166.0956757 | 485.317 |
| Biomarker b30 | 3-Fluorocatechol | C₆FH₅O₂ | 146.0600839 | 531.627 |
| Biomarker b31 | Thymine | C₅H₆N₂O₂ | 144.0761483 | 532.091 |
| Biomarker b32 | Sulfoacetaldehyde | C₂H₄O₄S | 123.9826417 | 769.939 |
| Biomarker b33 | 2-Oxobutanoate | C₄H₆O₃ | 103.0389661 | 114.685 |
| Biomarker b34 | N-Formiminoglycine | C₃H₆N₂O₂ | 103.0500104 | 485.3545 |
| Biomarker b35 | 2,4-Diene-VPA | C₈H₁₂O₂ | 141.0908185 | 634.193 |
| Biomarker b36 | 3-Dimethylallyl-4-hydroxybenzoate | C₁₂H₁₄O₃ | 206.0948611 | 531.681 |
| Biomarker b37 | Aphidicolin | C₂₀H₃₄O₄ | 356.2792855 | 666.068. |

In another preferred embodiment, the set comprises biomarkers b1 to b37.

In another preferred embodiment, the set comprises biomarker bm and one or more biomarker(s) selected from a subset X, wherein the subset X consists of biomarkers b1 to b(m-1) and biomarkers b(m+1) to b37, m is an integer and 1≤m≤37, preferably 1≤m≤7, and more preferably 1≤m≤5.

In another preferred embodiment, m is 1, 2, 3, 4, 5, 6, 7, 8, 9, 10, 11, 12, 13, 14, 15, 16, 17, 18, 19, 20, 21 , 22, 23, 24, 25, 26, 27, 28, 29, 30, 31, 32, 33, 34, 35, 36, or 37;
wherein the biomarkers b1 to b(m-1) do not exist when m is 1;
the biomarkers b1 to b(m-1) represent biomarker b1 when m is 2;
the subset X consists of biomarkers b1 to b(m-1) and biomarkers b(m+1) to b37 when m is any integer from 3 to 35;
the biomarkers b(m+1) to b37 represent biomarker b37 when m is 36;
the biomarkers b(m+1) to b37 do not exist when m is 37.

In another preferred embodiment, the set comprises biomarkers b1 to b7 and one or more biomarker(s) selected from a subset Y, wherein the subset Y consists of biomarkers b8 to b37.

In another preferred embodiment, the set comprises biomarker(s) b1 to b5 and one or more biomarkers selected from subset Z, wherein subset Z consists of biomarkers b6 to b37.

In another preferred embodiment, the set comprises biomarkers b1 and b5.

In another preferred embodiment, the set comprises biomarker(s) selected from the group consisting of:
biomarker b1, biomarker b2, biomarker b3, biomarker b4, biomarker b5, biomarker b6, biomarker b7, and the combination thereof.

In another preferred embodiment, the set comprises biomarker b1 and one or more biomarker(s) selected from a subset A, wherein the subset A consists of biomarkers b2 to b7.

In another preferred embodiment, the set comprises biomarker b2 and one or more biomarker(s) selected from a subset B, wherein the subset B consists of biomarker b1 and biomarkers b3 to b7.

In another preferred embodiment, the set comprises biomarker b3 and one or more biomarker(s) selected from a subset C, wherein the subset C consists of biomarkers b1 to b2 and biomarkers b4 to b7.

In another preferred embodiment, the set comprises biomarker b4 and one or more biomarker(s) selected from a subset D, wherein the subset D consists of biomarkers b1 to b3 and biomarkers b5 to b7.

In another preferred embodiment, the set comprises biomarker b5 and one or more biomarker(s) selected from a subset E, wherein the subset E consists of biomarkers b1 to b4 and biomarkers b6 to b7.

In another preferred embodiment, the set comprises biomarker b6 and one or more biomarker(s) selected from a subset F, wherein the subset F consists of biomarkers b1 to b5 and biomarker b7.

In another preferred embodiment, the set comprises biomarker b7 and one or more biomarker(s) selected from a subset G, wherein the subset G consists of biomarkers b1 to b6.

In another preferred embodiment, the set comprises biomarkers b1 to b7.

In another preferred embodiment, the set comprises biomarkers selected from the group consisting of:
biomarker b1, biomarker b2, biomarker b3, biomarker b4, biomarker b5, and the combination thereof.

In another preferred embodiment, the set comprises biomarkers b1 to b5.

In another preferred embodiment, the set comprises biomarkers b1 and b5.

In another preferred embodiment, the set comprises biomarker(s) selected from the group consisting of: biomarker b1, biomarker b3, biomarker b4, biomarker b6, and the combination thereof.

In another preferred embodiment, the biomarker or biomarkers set is from samples of blood, plasma, or serum.

In another preferred embodiment, when compared with a reference value, one or more biomarker(s) selected from a subset H is (are) increased indicating that the test subject is in the risk of CHD or has CHD,
wherein the subset H includes biomarkers b2, b4, b5, b6, b8, b10, b15, b16, b17, b18, b21, b22, b23, b24, b26, b27, b31, b32, b33, b34, and b35.

In another preferred embodiment, the subset H includes biomarkers b2, b4, b5, and b6.

In another preferred embodiment, the subset H includes biomarkers b2, b4 and b5.

In another preferred embodiment, the subset H includes biomarker b5.

In another preferred embodiment, when compared with a reference value, one or more biomarker(s) selected from a subset K is(are) decreased, indicating that the test subject is in the risk of CHD or has CHD,
wherein the subset K includes biomarkers b1, b3, b7, b9, b11, b12, b13, b14, b19, b20, b25, b28, b29, b30, b36 and b37.

In another preferred embodiment, the subset K includes biomarkers b1, b3, and b7.

In another preferred embodiment, the subset K includes biomarkers b1 and b3.

In another preferred embodiment, the subset K includes biomarker b1.

In another preferred embodiment, each biomarker is identified by mass spectrometry, preferably chromatography-mass spectrometry, such as gas chromatography-mass spectrometry (GC-MS) or liquid chromatography-mass spectrometry (LC-MS).

In another preferred embodiment, the set is used for the evaluation on the risk of CHD or CHD diagnosis in a test subject.

In another preferred embodiment, said the evaluation on the risk of CHD in a test subject includes early screening for CHD

In a second aspect, the present invention provides a reagent combination for evaluating or diagnosing a risk of CHD, the reagent combination comprises a reagent for detecting each biomarker in the set according to the first aspect of the present invention.

In another preferred embodiment, the reagent comprises a substance for detecting each biomarker in the set according to the first aspect of the present invention by mass spectrometry.

In a third aspect, the present invention provides a kit comprising the set according to the first aspect of the present invention and/or the reagent combination according to the second aspect of the present invention.

In another preferred embodiment, each biomarker in the set according to the first aspect of the present invention is used as a standard.

In another preferred embodiment, the kit further comprises a specification describing a reference dataset (for example, the data shown in Table 7) of the levels of each biomarker in the set according to the first aspect of the present invention from CHD patients and/or healthy controls.

In a fourth aspect, the present invention provides a use of the set according to the first aspect of the present invention and/or the reagent combination according to the second aspect of the present invention for preparing a kit, the kit is used for the evaluation on the risk of CHD or CHD diagnosis in a test subject.

In another preferred embodiment, the evaluation or diagnosis includes the steps of:
(1) providing a sample from a test subject and detecting the level of each biomarker in the set according to the first aspect of the present invention in the sample;
(2) comparing the level detected in step (1) with a reference dataset or a reference value (such as a reference value of a healthy control);
preferably, the reference dataset includes the levels of each biomarker in the set according to the first aspect of the present invention from CHD patients and healthy controls.

In another preferred embodiment, the sample is selected from the group consisting of: blood, plasma, and serum.

In another preferred embodiment, the step of comparing the level detected in step (1) with a reference dataset further comprises a step of establishing a multivariate statistical model to output the possibility of developing the disease, and preferably, the multivariate statistical model is a random forest model.

In another preferred embodiment, the subject is determined as the one who is in the risk of CHD or has CHD if the possibility of developing the disease is ≥ 0.5.

In another preferred embodiment, when compared with a reference value, one or more biomarker(s) selected from a subset H is (are) increased indicating that the test subject is in the risk of CHD or has CHD,
wherein the subset H includes biomarkers b2, b4, b5, b6, b8, b10, b15, b16, b17, b18, b21, b22, b23, b24, b26, b27, b31, b32, b33, b34, and b35.

In another preferred embodiment, the subset H includes biomarkers b2, b4, b5, and b6.

In another preferred embodiment, the subset H includes biomarkers b2, b4 and b5.

In another preferred embodiment, the subset H includes biomarker b5.

In another preferred embodiment, when compared with a reference value, one or more biomarker(s) selected from a subset K is(are) decreased indicating that the test subject is in the risk of CHD or has CHD,
wherein the subset K includes biomarkers b1, b3, b7, b9, b11, b12, b13, b14, b19, b20, b25, b28, b29, b30, b36 and b37.

In another preferred embodiment, the subset K includes biomarkers b1, b3, and b7.

In another preferred embodiment, the subset K includes biomarkers b1 and b3.

In another preferred embodiment, the subset K includes biomarker b1.

In another preferred embodiment, the level of each biomarker is detected by mass spectrometry, preferably by chromatography-mass spectrometry, such as gas chromatography-mass spectrometry (GC-MS) or liquid chromatography-mass spectrometry (LC-MS).

In another preferred embodiment, the method further comprises a step of processing the sample prior to step (1).

In a fifth aspect, the present invention provides a method for the evaluation on a risk of CHD or CHD diagnosis in a test subject, comprising the steps of:
(1) providing a sample from a test subject and detecting the level of each biomarker in the set according to the first aspect of the present invention in the sample;
(2) comparing the level detected in step (1) with a reference dataset or a reference value (such as a reference value of a healthy control);
preferably, the reference dataset includes the levels of each biomarker in the set according to the first aspect of the present invention from CHD patients and healthy controls.

In a sixth aspect, the present invention provides a method for screening a candidate compound for the treatment of CHD, comprising the steps of:
(1) in a test group, administering a test compound to a test subject, and detecting the level VI of each biomarker in the set according to the first aspect of the present invention from the sample of the subject in the test group; and in a control group, administering a blank control (including a vehicle) to a test subject, and detecting the level V2 of each biomarker in the set of the first aspect of the present invention from the sample of the subject in the control group;
(2) comparing the level VI with the level V2 detected in the previous step to determine whether the test compound is a candidate compound for the treatment of CHD

In another preferred embodiment, the test subject is a CHD patient.

In another preferred embodiment, if the level VI of one or more biomarker(s) selected from a subset H is significantly lower than the level V2, indicating that the test compound is a candidate compound for the treatment of CHD,
wherein the subset H includes biomarkers b2, b4, b5, b6, b8, b10, b15, b16, b17, b18, b21, b22, b23, b24, b26, b27, b31, b32, b33, b34, and b35.

In another preferred embodiment, the subset H includes biomarkers b2, b4, b5, and b6.

In another preferred embodiment, the subset H includes biomarkers b2, b4 and b5.

In another preferred embodiment, the subset H includes biomarker b5.

In another preferred embodiment, the "significantly lower than" means the ratio of level V1 to level V2 is ≤ 0.8, preferably ≤ 0.6.

In another preferred embodiment, if the level VI of one or more biomarker(s) selected from a subset K is significantly higher than the level V2, indicating that the test compound is a candidate compound for the treatment of CHD,
wherein the subset K includes biomarkers b1, b3, b7, b9, b11, b12, b13, b14, b19, b20, b25, b28, b29, b30, b36 and b37.

In another preferred embodiment, the subset K includes biomarkers b1, b3, and b7.

In another preferred embodiment, the subset K includes biomarkers b1 and b3.

In another preferred embodiment, the subset K includes biomarker b1.

In another preferred embodiment, the "significantly higher than" means the ratio of level VI to level V2 is ≥ 1.2, preferably ≥ 1.5.

In a seventh aspect, the present invention provides a use of the set according to the first aspect of the present invention and/or the reagent combination according to the second aspect of the present invention for screening a candidate compound for the treatment of CHD and/or evaluating the therapeutic effect of a candidate compound on CHD

In an eighth aspect, the present invention provides a method of establishing a mass spectrometry model for evaluating a risk of CHD or diagnosing CHD, comprising a step of identifying a differentially expressed substance in a blood sample between a patient and a healthy control, wherein the differentially expressed substance comprises one or more biomarker(s) in the set according to the first aspect of the present invention.

It should be understood that, in the present invention, each of the technical features specifically described above and below (such as those in the Examples) can be combined with each other, thereby constituting new or preferred technical solutions, which needs not be described one by one, due to space limitations.

### Description of Drawings

Figure 1 shows a two-dimensional PCA scores plot of the QC sample and the test sample. Each dot in the plot represents a sample, wherein the black rhombus dots represent test samples and white circles dots represent QC samples.
Figure 2 shows a cloud plot of 2588 metabolites. The gray line of background in the graph represents the total ion chromatogram for each sample; each bubble represents one metabolite, and the position of the bubble represents the retention time (x-coordinate) and mass-to-charge ratio (y-coordinate) of the metabolites. The upper half of the graph shows the increased intensity of 808 metabolites increased (fold change > 1.2) in CHD patients; the lower half shows the decreased intensity of 793 (fold change < 0.8) metabolites decreased in CHD patients.
Figure 3 shows a PCA scores plot of 40 CHD patients and 43 healthy controls. Each dot in the plot represents a sample, wherein the black circles dots represent healthy controls and white rectangle dots represent CHD patients.
Figure 4 shows a three-dimensional (3D) PLS-DA plot of plasma sample. The black rhombus dots and the white rectangle dots represent CHD patients and healthy controls respectively. PC1 (42.86%), PC2 (10.72%), and PC3 (13.68%) in PLS-DA shows that the metabolic profiling of plasma samples from CHD patients and healthy controls have significant difference. The model is established using 10-fold cross-validation, wherein R2 is 61.26% and Q2 is 48.32%.
Figure 5 shows a R2 profile of the permutation test for the PLS-DA model of plasma samples. The R2 value (thick vertical bar on the right) of the model is significantly separated from the H0 random classifier permutation distribution (a series of vertical lines on the left, N = 1000). Therefore, the probability of random occurrence of this model is less than 0.001.
Figure 6 shows an S plot containing model covariance (x-axis) and model dependence (y-axis) of the PLS-DA model in the scatter plot. The white triangles represent the metabolites with VIP>1 and the black triangles represent the metabolites with VIP≤1.
Figure 7 shows a volcano plot containing the statistical test results (y-axis: -log (q value)) and the amplitude of variation (Log2 (FC)) of the metabolites in the scatter plot. The dots on either side of the dashed line represent metabolites with q value < 0.05, FC > 1.2 or FC < 0.8. Dots in the middle of the dashed line represent metabolites with q-value <0.05 and FC values between 0.8 and 1.2. Dots below the horizontal line represent metabolites with q value < 0.05.
Figure 8 shows a Venn diagrams obtained by the results of the volcano plot and the S plot. The results show that 83 metabolites are significantly changed in CHD patients. The ellipse on the left represents 1140 metabolites significantly changed (q value < 0.05, FC > 1.2 or FC < 0.8) in the volcano plot. The ellipse on the right represents 138 metabolites significantly changed (VIP>1) in the S-map. The overlap region of the two ellipses represents 83 metabolites satisfied both the q value < 0.05, FC > 1.2 or FC < 0.8, and VIP >1.
Figure 9 shows the correlation between the intensities of seven potential biomarkers. Each rectangle represents a specific metabolite. Levels of the metabolites on the left side of the vertical line are increased in CHD patients and levels of the metabolites on the right side of the vertical line are significantly decreased in CHD patients. The lines connecting the metabolites represent a significant correlation (Spearman correlation adjustment p value < 0.05). The solid lines represent positive correlation and the dotted lines represent negative correlation.
Figure 10 shows a receiver operating characteristic (ROC) analysis curve of five identified potential plasma biomarkers in a validation set.
Figure 11 shows a receiver operating characteristic (ROC) analysis curve of the five metabolite biomarkers identified by random forest classifier in a training set.
Figure 12 shows a ROC curve of the five metabolite biomarkers in the training set, respectively.
Figure 13 shows a box plot of abundance levels of the five metabolite biomarkers in CHD patients and healthy control samples of the training set, respectively.
Figure 14 shows a ROC curve of the binding of two metabolite biomarkers in the validation set.

### Embodiments for Carrying Out the Present Invention

After the extensive and intensive research, the inventor firstly discovered a coronary heart disease (CHD) biomarker. Specifically, the present invention has found a set of biomarkers comprising a variety of biomarkers for coronary heart disease which can be used for the evaluation on the risk of CHD or CHD diagnosis in a test subject. The biomarkers have an advantage of high sensitivity and high specificity and have important application value.

### Term

The terms used in the present invention have the meanings as commonly understood by the ordinary skilled in the relevant art. However, for a better understanding of the present invention, some definitions and related terms are explained as follows:
According to the present invention, the term "coronary heart disease", also know as "coronary heart disease (CHD)" or "coronary artery disease (CAD)" refers to a group of diseases with symptoms including stable angina pectoris, unstable angina pectoris, myocardial infarction and sudden coronary death and so on. In the present invention, patients with CHD are diagnosed by coronary angiography techniques.

According to the present invention, the term "a set of biomarkers" refers to a combination of one biomarker, two or more biomarkers.

According to the present invention, mass spectrometry (MS) can be classified into ion traps mass spectrometry, quadrupole mass spectrometry, orbitrap mass spectroscopy and time-of-flight mass spectrometry, and the deviations are 0.2 amu, 0.4 amu, 3 ppm and 5 ppm, respectively. In the present invention, the MS data is obtained using orbitrap mass spectrometry.

According to the present invention, the levels of the biomarkers are indicated by peak intensity in MS.

According to the present invention, it is known in the art that the mass-to-charge ratio (m/z) and retention time (RT) have the same meaning. In the present invention, the unit of m/z is amu, which refers to atomic mass unit, also named as Dalton. Dalton represents the standard unit of matter on atomic or molecular scale (atomic mass). One uniform atomic mass unit is equivalent to 1 g/mol. It is defined as one-twelfth of the atomic mass of carbon-12. The allowable mass resolution (error) in the identification of the metabolites in the present invention is 10 ppm, wherein ppm is one part per million. For example, the exact mass of the isotope of metabolite A = 118 Da, the mass measured by instrument = 118.001 Da; the deviation = 0.001 amu; the error [deviation / exact mass × 106] = 8.47 ppm.

It is clear to the skilled in the art that the values of m/z and retention time will vary within a certain extent when using different detection methods or LC-MS instruments. For example, m/z can be varied in the range of ±3.00, or ±2.00, or ±1.00; retention time can be varied in the range of ±60 seconds, or ±45 seconds, or ±30 seconds, or ±15 seconds.

According to the present invention, a reference set refers to a training set.

According to the present invention, it is known in the art that a training set and a verification set have the same meaning. In one embodiment of the present invention, the training set refers to a set of the levels of biomarkers in the biological samples from CHD patients and healthy controls. In one embodiment of the present invention, the verification set refers to a dataset used to test the performance of the training sets. In one embodiment of the present invention, the levels of biomarkers can be indicated as absolute values or relative values according to the method of determination. For example, when mass spectrometry is used to determine the levels of biomarkers, the intensity of a peak can represent the levels of biomarkers, which is a level of relative value; when PCR is used to determine the levels of biomarkers, the copy number of gene or gene segment can represent the levels of biomarkers.

In one embodiment of the present invention, the reference value refers to a reference value or a normal value of a healthy control. It is clear to the skilled in the art that the range of normal value (absolute value) of each biomarker can be obtained with test and calculation method when the number of samples is large enough. Therefore, when the levels of biomarkers are detected using methods other than mass spectrometry, the absolute values of the levels of these biomarkers can be directly compared with normal values, thereby evaluating the risk of CHD, as well as diagnosing or early diagnosing CHD. Optionally, statistical methods can also be used.

According to the present invention, the term "a biomarker", also known as "a biological marker", refers to a measurable indicator of a biological state of a subject. Such biomarkers can be any substance in the subject, as long as they are in relation with a particular biological state (e.g., disease) of the test subject, for example, nucleic acid marker (e.g., DNA), protein marker, cytokine marker, chemokine marker, carbohydrate marker, antigen marker, antibody marker, species marker (species/genus marker), and functional markers(KO/OG marker). Biomarkers are often measured and evaluated to examine normal biological processes, pathogenic processes, or pharmacological responses to a therapeutic intervention, and are useful in many scientific fields.

According to the present invention, the term "subject" refers to an animal, in particular a mammal, such as a primate, most preferably a human.

According to the present invention, the term "plasma" refers to the fluid component of whole blood. Depending on the used separation method, plasma may be completely free of cellular components, or may contain various amounts of platelets and/or small amounts of other cellular components.

According to the present invention, terms such as "a", "an" and "the" are not intended to refer to a singular entity but include a generic class of which a specific embodiment may be used for illustration.

It should be noted that the explanations of the terms are provided herein only for the skilled in the art can better understand the present invention, but not intended to limit the present invention.

### The main advantages of the present invention are:

(a) The present invention uses plasma metabolites as biomarkers for early screening, prediction, prevention and treatment of CHD, which has advantages of high sensitivity and high specificity, and has important application value.
(b) The use of plasma as the samples of biomarkers detection has advantages of convenient material acquisition, simple operation procedures and continuous detection in vitro.
(c) The biomarkers of the present invention can be used for early screening, prediction, prevention and treatment of CHD with the characteristic of good repeatability.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The biomarker b5 in the present patent application corresponds to the biomarker 18 in the PCT patent application (Application No. PCT/CN2015/087664).

### Example 1

### 1. Materials and Methods

### 1.1 Sample Collection

83 volunteers were recruited from Guangdong Provincial People's Hospital. Among them, 40 volunteers were CHD patients diagnosed by angiography and other 43 volunteers were healthy controls. The blood samples from volunteers were collected using a Vacuette EDTA blood collection tube and centrifuged at 14000 g for 10 minutes at 4°C to obtain plasma samples, which were stored at -80°C until use. (The volunteer's clinical information is listed in Table 1). This experiment was approved by the Institutional Review Board of Shenzhen Huada Genomics Institute. Before collecting samples, written consents were obtained from all volunteers.

### 1.2 Materials and Reagents

Formic acid (HPLC-grade) was purchased from Thermo Fisher Scientific (Loughborough, UK). Pure water used in the experiment was prepared using a Milli-Q Ultra-pure water system (Millipore, Billerica, MA, USA). The analytical column used was an Agilent ZORBAX ODS C18 column (Agilent Technologies, Santa Clara, CA, USA) (150 mm × 2.1 mm, 3.5 µm).

### 1.3 Sample Preparation

In order to extract metabolites with low molecular weight (< 1500 daltons) from plasma samples, the samples were prepared using a method similar to the previously reported method(Luan H, Meng N, Liu P, Fu J, Chen X, Rao W, Jiang H, Xu X, Cai Z, Wang J. Untargeted metabolomics and lipidomics LC-MS data from maternal plasma of 180 healthy pregnant women. Gigascience [Internet]. 2015; 4: 16-19., Incorporated herein by reference). Prior to the experiment, all plasma samples were thawed on ice. From each plasma samples, 10 µL was taken respectively and mixed to obtain a "quality control" (QC) sample. As for plasma samples, 100 µL samples were taken and mixed with 200 µL methanol to precipitate the protein. The mixture was then centrifuged at 14000 g for 10 minutes at 4°C. The supernatants were transferred to a 1.5 mL polypropylene tube for the following metabolomic profiling analysis experiments.

### 1.4 HPLC-MS Experiments

LTQ OrbitrapVelos instrument (Thermo Fisher Scientific, MA, USA) at 30,000 resolution was coupled to a Shimadzu Prominence HPLC system (Shimadzu Scientific Instruments, Kyoto, Japan). All samples were analyzed in positive ion modes. The cone voltage was set as 4.5KV and the capillary temperature was set as 350 °C. The mass scanning range was 50-1500m/z. The flow rates of nitrogen sheath gas and auxiliary gas were set as 30 L/min and 10 L/min. The HPLC-MS system was operated in binary gradient mode. Solvent A was 0.1% (v/v) formic acid/water and solvent B was 0.1% (v/v) formic acid/methanol. The gradient of mobile phase was as follows: 0 min, 5% B; 5 min, 5% B; 8 min, 100% B; 9 min, 100% B; 18 min, 5% B; 20 min, 5% B. The flow rate was 0.2 mL/min. Five QC samples were injected at the beginning of the experiment to ensure system equilibrium. Meanwhile, one QC sample was injected per five test samples to check the stability of the system throughout the experiment.

### 1.5 Data Processing and Statistical Analysis

The pretreatment of HPLC-MS data includes the following procedures: peak picking, peak grouping, retention time correction, second peak grouping, and annotation of isotopes and adducts (Lu K, Knutson CG, Wishnok JS, Fox JG, Tannenbaum SR. Serum metabolomics in a Helicobacter hepaticus mouse model of inflammatory bowel disease reveal important changes in the microbiome, serum peptides, and intermediary metabolism. J Proteome Res. ACS Publications; 2012;11:4916-4926., incorporated herein by reference). The raw data files obtained by LC-MS was converted into mzXML format and then processed by the XCMS (Smith CA, Want EJ, O'Maille G, Abagyan R, Siuzdak G XCMS: processing mass spectrometry data for metabolite profiling using nonlinear peak alignment, matching, and identification. Anal Chem. ACS Publications; 2006;78:779-787., incorporated herein by reference) and CAMERA (Kuhl C, Tautenhahn R, Bottcher C, Larson TR, Neumann S. CAMERA: an integrated strategy for compound spectra extraction and annotation of liquid chromatography/mass spectrometry data sets. Anal Chem. ACS Publications; 2011;84:283-289., incorporated herein by reference) toolkit in R software (V3.1.1). In addition, the intensity of each peak was recorded and a three dimensional matrix containing arbitrarily ionic peak indices (retention time and m/z pairs), sample names (observed value), and ion intensity information (variables) was generated.

Peaks with missing values over 80% (ion intensity = 0) and the isotopic ions existing in both CHD and a healthy control group was further removed from the obtained matrix in order to obtain a consistent result. The peak intensity of all the experimental samples was normalized to the peak intensity of the QC sample using Robust Loess signal correction (R-LSC) method based on the quantitative analysis of a standard biological quality control sample (QC sample) together with the real plasma samples (Dunn WB, Broadhurst D, Begley P, Zelena E, Francis-McIntyre S, Anderson N, Brown M, Knowles JD, Halsall A, Haselden JN. Procedures for large-scale metabolic profiling of serum and plasma using gas chromatography and liquid chromatography coupled to mass spectrometry. Nat Protoc. Nature Publishing Group; 2011; 6: 1060-1083., incorporated herein by reference), thereby ensuring the quality of metabolic profiles. The relative standard deviation (RSD) values of metabolites in the QC samples were set at a threshold of 30% which was accepted as a standard for the assessment of repeatability in metabolomics data sets (Dunn WB, Wilson ID, Nicholls AW, Broadhurst D. The importance of experimental design and QC samples in large-scale and MS-driven untargeted metabolomic studies of humans. Bioanalysis. Future Science; 2012; 4: 2249-2264., incorporated herein by reference).

The nonparametric univariate method (Mann-Whitney-Wilcoxon test) was performed to search the significantly changed metabolites in the CHD patients compared with healthy controls. The obtained results were corrected using the false discovery rate (FDR) to ensure that the peaks of metabolites were repeatedly detected (Ling XB, Cohen H, Jin J, Lau I, Schilling J. FDR made easy in differential feature discovery and correlation analyses. Bioinformatics. Oxford Univ Press; 2009;25:1461-1462., incorporated herein by reference). Multivariate statistical analysis (PCA, PLS-DA) method was performed to discriminate CHD patient samples and control subject samples. Based on the 10-fold cross-validation PLS-DA model, the variable importance in the projection (VIP) threshold was set as 1 to obtain a large number of metabolites responsible for the difference in the metabolic profile of CHD patients and control subjects. The PLS-DA model was validated at one-way level using FDR test in the R Software Statistical Toolkit (p < 0.05). Three-dimensional PLS-DA was also implemented to show the differences between CHD patient samples and control subject samples (Triba MN, Moyec L Le, Amathieu R, Goossens C, Bouchemal N, Nahon P, Rutledge DN, Savarin P. PLS/OPLS models in metabolomics: the impact of permutation of dataset rows on the K-fold cross-validation quality parameters. Mol Biosyst. Royal Society of Chemistry; 2015; 11: 13-19., incorporated herein by reference). Spearman correlation analysis was implemented to analyze the correlations of the significantly changed plasma metabolites and clinical phenotype data in CHD patients and control subjects. In addition, receiver operating characteristic (ROC) analysis was used to evaluate diagnostic capabilities of identified potential biomarkers (Ndrepepa G, Braun S, Kastrati A, Schömig A. Area under ROC curve, sensitivity, specificity of N-terminal probrain natriuretic peptide in predicting mortality in various subsets of patients with ischemic heart disease. Clin Res Cardiol. Springer; 2007;96:763-765., incorporated herein by reference).

### 1.6 Metabolite Identification and Verification

Metabolites were identified by comparing the exact molecular mass data (m/z) of metabolites in the samples with the online HMDB database (http://www.hmdb.ca)(Wishart DS, Tzur D, Knox C, Eisner R, Guo AC, Young N, Cheng D, Jewell K, Arndt D, Sawhney S. HMDB: the human metabolome database. Nucleic Acids Res. Oxford Univ Press; 2007;35:D521-D526.; Wishart DS, Knox C, Guo AC, Eisner R, Young N, Gautam B, Hau DD, Psychogios N, Dong E, Bouatra S. HMDB: a knowledgebase for the human metabolome. Nucleic Acids Res. Oxford Univ Press; 2009;37:D603-D610.; Wishart DS, Jewison T, Guo AC, Wilson M, Knox C, Liu Y, Djoumbou Y, Mandal R, Aziat F, Dong E. HMDB 3.0-the human metabolome database in 2013. Nucleic Acids Res. Oxford Univ Press; 2012;gks1065., incorporated herein by reference) and KEGG database (www.genome.jp/kegg/)(Kanehisa M, Goto S. KEGG: kyoto encyclopedia of genes and genomes. Nucleic Acids Res. Oxford Univ Press; 2000;28:27-30.; Kanehisa M, Goto S, Sato Y, Kawashima M, Furumichi M, Tanabe M. Data, information, knowledge and principle: back to metabolism in KEGG Nucleic Acids Res. Oxford Univ Press; 2014; 42: D199-D205., incorporated herein by reference) Metabolites would be identified if the mass difference between observations and database values was less than 10 ppm. The molecular formula of the metabolites will be further verified by isotope distribution measurements. Those significantly changed metabolites were validated and confirmed by comparing the MS/MS spectra and retention times of the purchased reference standards (Chen J, Zhao X, Fritsche J, Yin P, Schmitt-Kopplin P, Wang W, Lu X, Häring HU, Schleicher ED, Lehmann R. Practical approach for the identification and isomer elucidation of biomarkers detected in a metabonomic study for the discovery of individuals at risk for diabetes by integrating the chromatographic and mass spectrometric information. Anal Chem. ACS Publications; 2008; 80: 1280-1289., incorporated herein by reference).

### 2. Experimental Results

### 2.1 Clinical information of Volunteers

The present invention analyzed the plasma samples (training sets) from 40 CHD patients and 43 healthy control subjects by untargeted metabolomics methods. The clinical information of all volunteers is shown in Table 1. A total of 13 biochemical indexes is included in the table including age (AGE), triglyceride (TRIG), lipoprotein (LPA), BMI, alanine aminotransferase (ALT), low density lipoprotein (LDLC), cholesterol (CHOL), high density lipoprotein (HDLC), aspartate aminotransferase (AST), apolipoprotein B (APOB), apolipoprotein A (APOA), albumin (ALB) and total protein (TP).

**Table 1 Clinical biochemical index information of the volunteers**

| | **CHD Patients** | **Healthy Control** | **P value** | **Number of volunteers^{#} (CHD/control)** |
|---|---|---|---|---|
| Sex (male/female) | 28/12 | 18/25 | -- | -- |
| AGE | 59.98(42-77) | 62.45(51-107) | 2.91E-01 | 40/38 |
| BMI | 24.8(16.44-30.48) | 24.75(17.63-50.32) | 9.59E-01 | 38/43 |
| TRIG | 1.77(0.57-5.96) | 1.11(0.45-3.49) | 1.33E-03 | 38/38 |
| LDLC | 2.8(0.93-5.7) | 3.49(2.49-5.47) | 1.03E-03 | 38/38 |
| CHOL | 4.6(2.87-8.11) | 5.62(3.86-7.88) | 5.71E-05 | 38/38 |
| HDLC | 1.04(0.67-1.62) | 1.4(0.82-2.31) | 1.20E-05 | 38/38 |
| APOB | 0.85(0.52-1.46) | 1.01(0.7-1.9) | 9.29E-04 | 31/38 |
| APOA | 1.09(0.67-1.63) | 1.23(0.67-2.01) | 5.18E-02 | 31/38 |
| LPA | 400.1(50.1-1580.84) | 149.65(11.17-933.54) | 2.01E-03 | 32/38 |
| ALB | 35.76(25.8-42) | 42.73(37-47.2) | 1.54E-15 | 38/39 |
| ALT | 30.07(13-76.4) | 26.79(2-59) | 3.70E-01 | 35/37 |
| TP | 64.38(52.3-79.1) | 74.92(67.3-86.9) | 7.93E-13 | 38/39 |
| AST | 25.75(16-61) | 25.78(17-52) | 9.88E-01 | 38/39 |

| | | | | |
|---|---|---|---|---|
| **# since not all the biochemical indexes were collected from all volunteers, the number of volunteers of CHD patients and healthy controls for each index is listed.** | | | | |

### 2.2 QC Sample Analysis

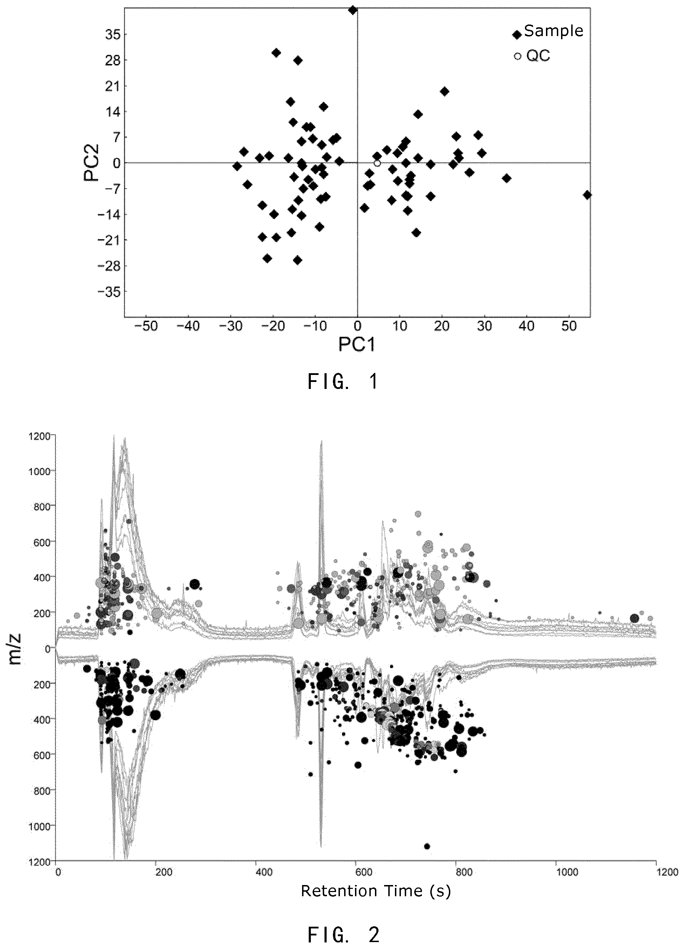

2588 metabolites (m/z, mass-to-charge ratio) were obtained from plasma samples using an untargeted HPLC-MS metabolome technology. QC samples were measured throughout the experiment to evaluate the stability and reproducibility of the dataset. The principal component analysis (PCA) was performed for QC samples and test samples to obtain a two-dimensional PCA score plot, which is shown in Figure 1. The results showed that the cluster formed by QC samples and test samples was not significantly dispersed, thereby the good stability and reproducibility of our current metabolomic data were confirmed.

### 2.3 Metabolome Data Analysis of Plasma

The inventors showed a cloud plot of all 2588 metabolites to illustrate the entire metabolome data profile (Figure 2). The results showed that compared with the healthy control group, 31.22% (808) of the metabolites were significantly increased in the plasma samples of CHD patients (fold change > 1.2), while 30.64% (793) of the metabolites were significantly creased in the plasma samples of CHD patients (fold change< 0.8).

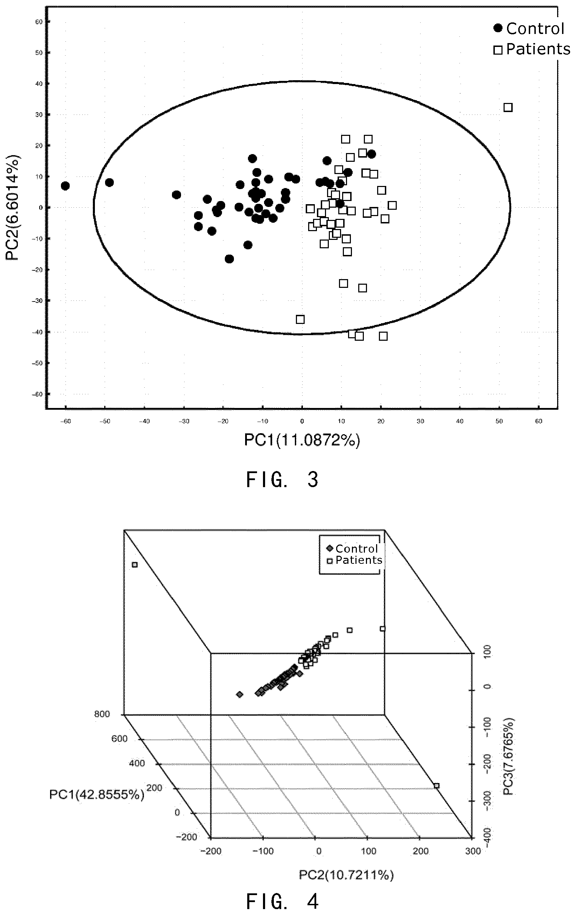

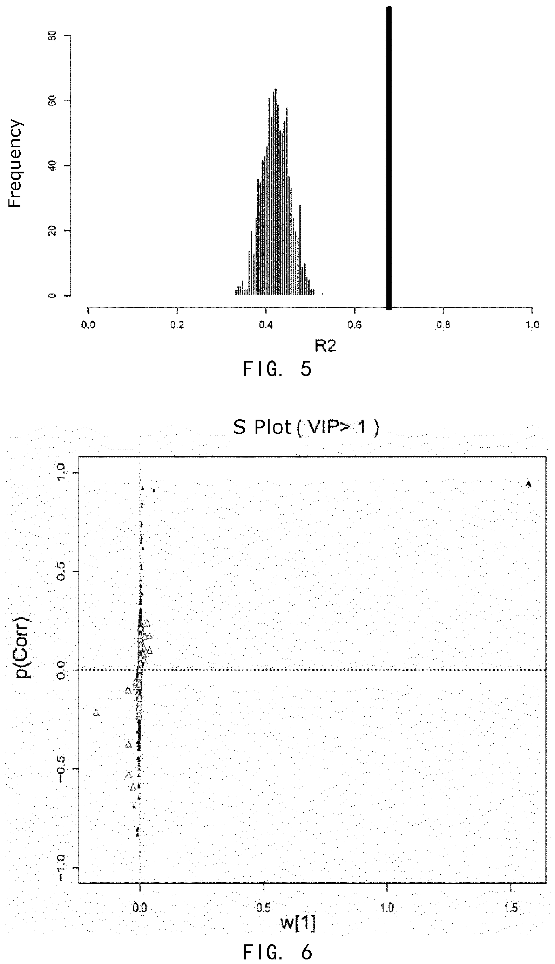

The inventors performed principal component analysis (PCA) on plasma samples from 40 CHD patients and 43 healthy control subjects and obtained a two-dimension PCA score plot of the samples (as shown in Figure 3) to evaluate the identification ability of the obtained 2588 metabolites. The results showed that the principal components PC1 and PC2 were 11.09% and 6.61%, and two independent clusters were formed in the CHD patients groups and control groups. In addition, three-dimensional partial least squares discriminant analysis (PLS-DA) was performed on the CHD patient group and the healthy control group. As shown in Figure 4, the principal components PC1, PC2 and PC3 were 42.86%, 10.72% and 7.68%, indicating that there was a significant difference between the two metabolome profiling. The PLS-DA model was constructed using 10-fold cross-validation and proved to be a good model using 3 components with a good predictive ability (accumulative total of R2 (X) = 61.26%, accumulative total of Q2 (X) = 48.32). The inventors further validated it using the permutation multivariate analysis (PERMANOVA) to avoid the over-fitting of the model. The R2 distribution profile of the permutation test for the PLS-DA model of plasma samples is shown in Figure 5. Three potential factors were used in the permutation model. The probability of random occurrence of this model is less than 0.001. The above results have verified that the metabolic profiling of CHD patients and controls are significantly different.

### 2.4 Identification of Metabolite Biomarker

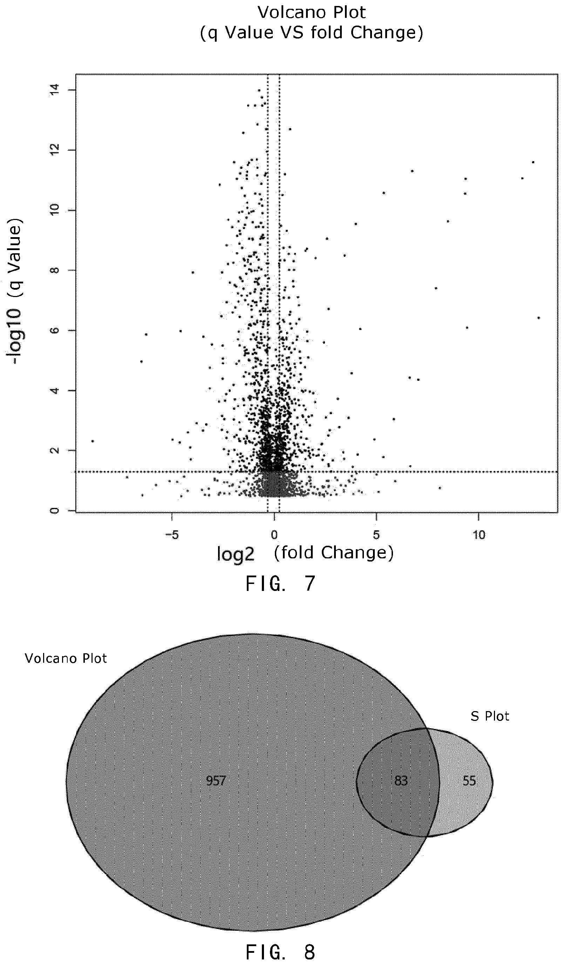

The inventors performed two types of analysis to identify the metabolites that are significantly changed in CHD patients and potential biomarkers of CHD. Firstly, VIP scores for all 2588 metabolites were obtained from the PLS-DA model and the supposed biomarkers were shown with S plot (Figure. 6). The covariance (x-axis) and correlation (y-axis) of the PLS-DA model were shown together in a scatter plot. The false discovery rate (q-value), fold change (FC), and mean intensity of each metabolite in CHD patients were normalized with the means of the same metabolite in healthy controls and then tested using a two-tailed Wilcoxon rank-sum test. A volcano plot was drawn with the size change and the statistical test results (Figure 7), which made it possible to quickly and intuitively identify metabolites with large fold change. The inventor drew Venn diagrams to integrate the results of S plot and the volcano plot(Fig. 8). Finally, 83 metabolites in the overlapping region met the following criterion (q value < 0.05, FC > 1.2 or FC < 0.8, VIP > 1).

To identify the potential biomarkers, the 83 significantly changed metabolites were aligned to the KEGG and HMDB databases (< 10 ppm). A total of 37 potential metabolites were identified, and their detailed information was listed in Table 2. 7 of the 37 metabolites including 2,3-dimethylmaleate, 2-nphthol, mthylitaconate, N-aetyl-D-glucosamine-6-phosphate, 1-nphthol, L-crnitine, and penylpyruvate were validated with purchased reference standards and which could be considered as a candidate potential biomarker for the diagnosis of CHD. Detail information of the seven metabolites was listed in Table 3. Among the seven potential biomarkers, 2,3-dimethylmaleate, 2-naphthol, methylitaconate and N-aetyl-D-glucosamine -6-phosphate were significantly increased (enrichment direction) in CHD patients with a fold change of 4.24,17.61,1.40 and 16.58, and q values of 3.88×10-9, 0.014, 3.14×10-11 and 2.85×10-10 respectively. The other three metabolites (1-naphthol, L-carnitine, and phenylpyruvate) were significantly decreased (enrichment direction) in CHD patients with a fold change of 0.27, 0.06 and 0.06 and q values of 0.0035, 0.0079 and 0.0198, respectively. Using the online tool Metabo Analyst 3.0 to analyze the relevant metabolic pathways, the following five pathways were identified (Table 4): metabolic pathway for nicotinate and nicotinamide, metabolic pathway for heterologous metabolic of cytochrome P450, phenylalanine pathway, biosynthesis pathways for tyrosine and tryptophan, metabolic pathway for phenylalanine, metabolic pathway for amino sugar and nucleotide sugar.

**Table 2 Annotation Information for 37 Potential Metabolite Biomarkers in the KEGG and HMDB Databases**

| **m/z** | **Name** | **RT(s)** | **FC** | **QValue** | **VIP** | **Molecular Formula** | **ppm** | **Adduct ion** | t **KEGG No.** |
|---|---|---|---|---|---|---|---|---|---|
| 496.3058791 | 20,26-Dihydroxyecdysone | 744.104 | 42.22277725 | 0.016537466 | 5.860576254 | C₂₇H₄₄O₈ | 4.555162899 | NAN | C16500 |
| 386.2897576 | Cortol | 662.962 | 0.177256317 | 1.17965E-08 | 1.127039967 | C₂₁H₃₆O₅ | 0.922283659 | NH⁴⁺ | C05482 |
| 372.0845309 | 10-Hydroxydihydrosanguinarin e | 684.837 | 677.6923435 | 9.06094E-12 | 1.152524356 | C₂₀H₁₅NO₅ | 0.831736433 | Na⁺ | C05247 |
| 337.215992 | Progesterone | 774.709 | 0.116855535 | 8.39054E-05 | 1.394324614 | C₂₁H₃₀O₂ | 6.981522154 | Na⁺ | C00410 |
| 330.2635353 | (9Z,11E)-(13S)-13-Hydroperoxyoctadeca-9,11-dienoic acid | 653.076 | 0.066128794 | 1.18451E-08 | 1.125637479 | C₁₈H₃₂O₄ | 1.112000483 | NH⁴⁺ | C04717 |
| 324.0458651 | N-Acetyl-D-glucosamine 6-phosphate | 541.3545 | 16.58021966 | 2.85283E-10 | 1.13430565 | C₈H₁₆NO₉P | 1.260022221 | Na⁺ | C00357 |
| 318.2999289 | Phytosphingosine | 656.764 | 0.125893793 | 2.88908E-06 | 1.364406697 | C₁₈H₃₉NO₃ | 1.075521125 | H⁺ | C12144 |
| 315.1211881 | p-Coumaroylagmatine | 145.31 | 0.055570756 | 0.002488346 | 11.72764284 | C₁₄H₂₀N₄O₂ | 2.157612793 | K⁺ | C04498 |
| 315.096019 | N6-(L-1,3-Dicarboxypropyl)-L-lysine | 142.1965 | 1.592761031 | 0.036647449 | 1.83995082 | C₁₁H₂₀N₂O₆ | 2.624066784 | K⁺ | C00449 |
| 311.0511119 | Dihydrokaempferol | 611.26 | 374.5030074 | 2.3627E-10 | 1.1413235 | C₁₅H₁₂O₆ | 5.18705781 | Na⁺ | C00974 |
| 310.0474631 | Indoleglycerol phosphate | 611.2495 | 6672.904368 | 2.5567E-12 | 1.144547978 | C₁₁H₁₄NO₆P | 8.26088368 | Na⁺ | C03506 |
| 309.0542143 | Medicarpin | 611.434 | 112.101097 | 5.01144E-12 | 1.139040566 | C₁₆H₁₄O₄ | 6.83977517 | K⁺ | C10503 |
| 274.9929078 | 2-Carboxy-2-hydroxy-8-carboxychromene | 91.3841 | 0.093092459 | 0.002158523 | 1.639179892 | C₁₁H₈O₆ | 9.906101971 | K⁺ | C14094 |
| 274.2736949 | Hexadecanoic acid | 656.679 | 0.587371243 | 0.013559472 | 7.944786552 | C₁₆H₃₂O₂ | 1.398491469 | NH⁴⁺ | C00249 |
| 268.1909849 | Xanthoxin | 571.68 | 136.591287 | 4.25694E-05 | 1.028619144 | C₁₅H₂₂O₃ | 1.068740737 | NH⁴⁺ | C13453 |
| 255.0378919 | 5-(3'-Carboxy-3'-oxopropyl)-4,6 -dihydroxypicolinate | 117.92 | 2.380334491 | 0.000212966 | 1.1251526 | C₁₀H₉NO₇ | 0.038231114 | NAN | C05656 |
| 249.1082592 | N-Acetyl-L-2-amino-6-oxopimelate | 117.643 | 2.34300363 | 7.79908E-08 | 1.034450246 | C₉H₁₃NO₆ | 0.645151037 | NH⁴⁺ | C05539 |
| 247.0927331 | 4-(L-Alanin-3-yl)-2-hydroxy-cis ,cis-muconate 6-semialdehyde | 146.379 | 2.089020606 | 4.15404E-06 | 1.166956456 | C₉H₁₁NO₆ | 1.192087644 | NH⁴⁺ | C04796 |
| 223.0935157 | 4-[(Hydroxymethyl)nitrosoamine ]-1-(3-pyridinyl)-1-butanone | 644.408 | 0.218596446 | 0.01310796 | 1.19187159 | C₁₀H₁₃N₃O₃ | 9.751791312 | NAN | C19563 |
| 203.1391048 | Ecgonine | 119.338 | 9.451089096 | 0.001669027 | 4.571128188 | C₉H₁₅NO₃ | 0.478055409 | NH⁴⁺ | C10858 |
| 170.0328456 | Creatine | 202.182 | 60.10103849 | 0.000899912 | 4.38910166 | C₄H₉N₃O₂ | 1.610273281 | K⁺ | C00300 |
| 167.0564801 | 7-Methylxanthine | 502.8765 | 0.574176323 | 0.00610874 | 1.356281558 | C₆H₆N₄O₂ | 0.774809813 | H⁺ | C16353 |
| 167.0315121 | Methylitaconate | 104.76 | 1.379822769 | 3.14059E-11 | 1.413895688 | C₆H₈O₄ | 0.245625265 | Na⁺ | C02295 |
| 166.0956757 | Cyromazine | 485.317 | 0.263651446 | 0.000927962 | 1.331115204 | C₆H₁₀N₆ | 6.132959094 | NAN | C14147 |
| 165.0548439 | Phenylpyruvate | 183.8775 | 0.061360622 | 0.019795195 | 1.6426491 | C₉H₈O₃ | 1.364400522 | H⁺ | C00166 |
| 162.1122492 | L-Carnitine | 114.494 | 0.060141963 | 0.007928564 | 4.09199533 | C₇H₁₅NO₃ | 1.366267104 | H⁺ | C00318 |
| 146.0600839 | 3-Fluorocatechol | 531.627 | 0.033234667 | 0.004338839 | 3.362862486 | C₆FH₅O₂ | 8.566078644 | NH⁴⁺ | C16472 |
| 145.0649552 | 1-Naphthol | 531.467 | 0.268318622 | 0.003501529 | 2.16639535 | C₁₀H₈O | 1.140369054 | H⁺ | C11714 |
| 145.0496085 | 2,3-Dimethylmaleate | 1156.85 | 4.237658146 | 3.88395E-09 | 1.595234889 | C₆H₈O₄ | 0.511934162 | H⁺ | C00922 |
| 144.0761483 | Thymine | 532.091 | 11.14033766 | 0.0190977 | 2.509501537 | C₅H₆N₂O₂ | 4.776910845 | NH⁴⁺ | C00178 |
| 144.0571958 | 2-Naphthol | 531.9175 | 17.60713144 | 0.013559472 | 2.711841051 | C₁₀H₈O | 2.215229197 | NAN | C11713 |
| 123.9826417 | Sulfoacetaldehyde | 769.939 | 6.838080583 | 0.002103838 | 1.268763864 | C₂H₄O₄S | 3.128414471 | NAN | C00593 |
| 103.0389661 | 2-Oxobutanoate | 114.685 | 14.94987497 | 0.035362506 | 1.20782616 | C₄H₆O₃ | 0.038724242 | H⁺ | C00109 |
| 103.0500104 | N-Formiminoglycine | 485.3545 | 1.58903394 | 0.028471865 | 1.381467531 | C₃H₆N₂O₂ | 1.891764033 | H⁺ | C02718 |
| 141.0908185 | 2,4-Diene-VPA | 634.193 | 7.166516022 | 0.049497492 | 1.078658653 | C₈H₁₂O₂ | 1.335800813 | H⁺ | C16656 |
| 206.0948611 | 3-Dimethylallyl-4-hydroxybenzoate | 531.681 | 0.751336614 | 2.13009E-05 | 1.031782125 | C₁₂H₁₄O₃ | 2.75016027 | NAN | C12458 |
| 356.2792855 | Aphidicolin | 666.068 | 0.249898458 | 3.64113E-10 | 1.019572906 | C₂₀H₃₄O₄ | 0.730441253 | NH⁴⁺ | C06088 |

**Table 3 Identification of potential biomarkers in CHD patients and controls**

| | **1-Naphthol** | **2-Naphthol** | **2,3-Dimethylmaleate** | **L-Carnitine** | **Phenylpyruvate** | **Methylitaconate** | **GlcNAc-6-P¹⁾** |
|---|---|---|---|---|---|---|---|
| **m/z** | 145.0649552 | 144.0571958 | 145.0496085 | 162.1122492 | 165.0548439 | 167.0315121 | 324.0458651 |
| **RT ²⁾(s)** | 531.467 | 531.9175 | 1156.85 | 114.494 | 183.8775 | 104.76 | 541.3545 |
| **FC³⁾** | 0.268 | 17.607 | 4.238 | 0.060 | 0.061 | 1.380 | 16.580 |
| **Q Value** | 0.004 | 0.014 | 0.000 | 0.008 | 0.020 | 0.000 | 0.000 |
| **VIP** | 2.166 | 2.712 | 1.595 | 4.092 | 1.643 | 1.414 | 1.134 |
| **Molecular Formula** | C₁₀H₈O | C₁₀H₈O | C₆H₈O₄ | C₇H₁₅NO₃ | C₉H₈O₃ | C₆H₈O₄ | C₈H₁₆NO₉P |
| **Adduct ion** | H⁺ | NAN | H⁺ | H⁺ | H⁺ | Na⁺ | Na⁺ |
| **AUC** | 0.686 | 0.652 | 0.870 | 0.666 | 0.641 | 0.910 | 0.893 |
| **Pathway** | ko00980⁴⁾ | ko00980⁴⁾ | ko00760⁵⁾ | ko04976⁶⁾ | Ko00360 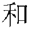 ko00400⁷⁾ | ko00760⁵⁾ | ko00520⁸⁾ |

| | | | | | | | |
|---|---|---|---|---|---|---|---|
| **1). N-Acetyl-D-glucosamine-6-phosphate;** **2). Retention time;** **3). fold change (CHD / control);** **4). ko00980, metabolic pathway for xenobioticof cytochrome P450;** **5). ko00760, metabolic pathway for nicotinate and nicotinamide;** **6). Ko04976, bile secretion pathway;** **7). Ko00360, Phenylalanine metabolic pathway; ko00400, biosynthesis pathways for phenylalanine, tyrosine and tryptophan;** **8). Ko00520, Metabolic pathway for amino sugar and nucleotide sugar.** | | | | | | | |

**Table 4 Pathways Associated with the 7 Significantly Changed Metabolites**

| **Pathway** | **Total Number ¹⁾** | **Matching Number ²⁾** | **P Value³⁾** | **Effect Value ⁴⁾** |
|---|---|---|---|---|
| metabolic pathway for nicotinate and nicotinamide | 44 | 2 | 0.0064714 | 0.05538 |
| metabolic pathway for xenobiotic of cytochrome P450 | 65 | 2 | 0.013818 | 0 |
| Biosynthesis pathways for phenylalanine, tyrosine and tryptophan | 27 | 1 | 0.076019 | 0 |
| metabolic pathway for phenylalanine | 45 | 1 | 0.1239 | 0.04875 |
| metabolic pathway for amino sugar and nucleotide sugar | 88 | 1 | 0.22976 | 0.01426 |

| | | | | |
|---|---|---|---|---|
| **1). The total number of metabolites in the pathway;** **2). The number of metabolites in the uploaded metabolite data matched with that in the pathway;** **3). Significance of the enriched metabolites in the pathway (Fisher's exact test);** **4). The effect value of the uploading metabolites in the pathway. The influence value refers to the importance of the metabolites position in the metabolic pathway network diagram (measured by the intermediary centrality of nodes in the network diagram).** | | | | |

### 2.5 Association analysis of potential metabolite biomarkers with clinical biochemical indexes

To evaluate the differences of 13 biochemical indexes between CHD and controls, the inventors performed a t-test and the results were shown in Table 1. The results showed that compared with the control group, LPA was significantly increased in the CHD patient group, while TRIG, LDLC, CHOL, HDL, APOB, ALB, and TP were significantly decreased. On the other hand, the levels of AST, ALT and APOA did not differ between the CHD patient group and the control group.

To evaluate the effect of covariates (clinical, biochemical factors such as age, BMI index) on the metabolic profiling in CHD patients, the inventors performed PERMANOVA analysis for the correlation between 7 potential biomarkers and 13 clinical biochemical indexes. The intensity of the most potential biomarkers was strongly correlated with the levels of ALB, TP, HDLC, CHOL, LDLC, TRIG and LPA but not with BMI, ALT, AGE, APOA, LDLC and AST. Interestingly, the 3 metabolites (2,3-dimethylmaleate, methylitaconate, and N-acetyl-D-glucosamine-6-phosphate) were significantly increased in CHD patients , however which were negatively correlated with ALB and TP levels and positively correlated with TRIG and LPA levels. Conversely, the 3 metabolites (1-naphthol, L-carnitine, and phenylpyruvate) decreased in CHD patients were positively correlated with ALB and TP levels and negatively correlated with TRIG and LPA levels. For example, the level of N-acetyl-D-glucosamine-6-phosphate was increased in CHD patients, which was significantly negatively correlated with TP and ALB (TP: p = 5.62E-05, ρ = -0.442; ALB: p = 6.15E -06, ρ= -0.490). In another example, the level of L-carnitine was significantly decreased in CHD patients, which was significantly positively correlated with TP and ALB (TP: P = 0.014, ρ = 0.278; ALB: P = 0.005, ρ = 0.315). The results of PERMANOVA analysis showed that these clinical indexes were related to the plasma metabolic profiling of patients with CHD

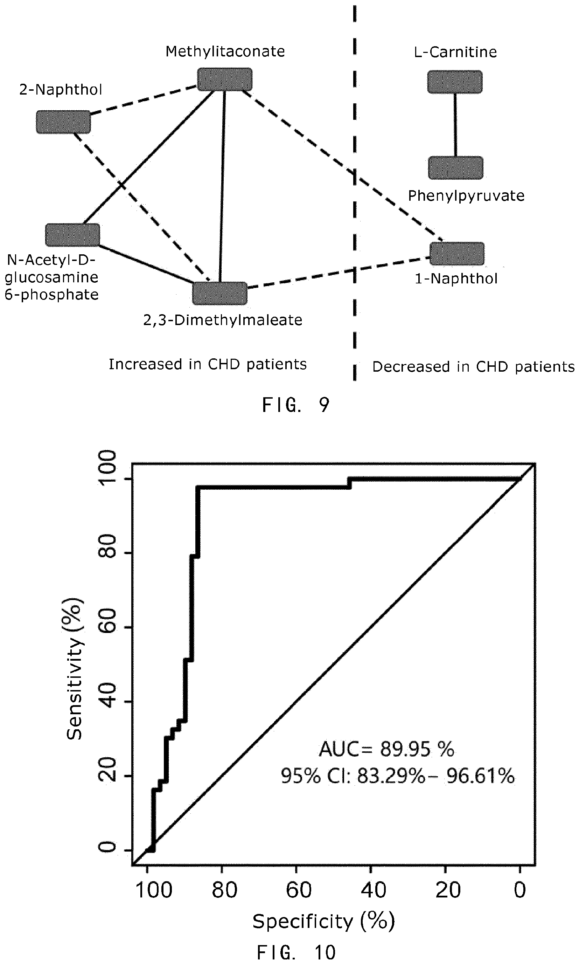

In addition, to evaluate the correlation of the seven potential biomarkers, the inventors performed a Spearman correlation analysis and the results were shown in Figure 9. The levels of 3 biomarkers (methylitaconate, N-acetyl-D- glycosamine-6-phosphate and 2,3-dimethylmaleate) were increased in CHD patients, and they are also significantly positively correlated with each other. On the other hand, the decreased levels of L-carnitine and phenylpyruvate were also positively correlated with each other in CHD patients, but they did not correlate with the other five biomarkers.

### 2.6 Receiver Operating Characteristics (ROC) Analysis of Potential Biomarkers

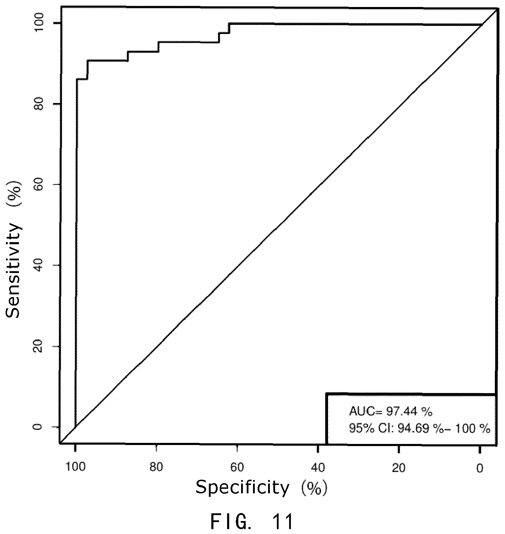

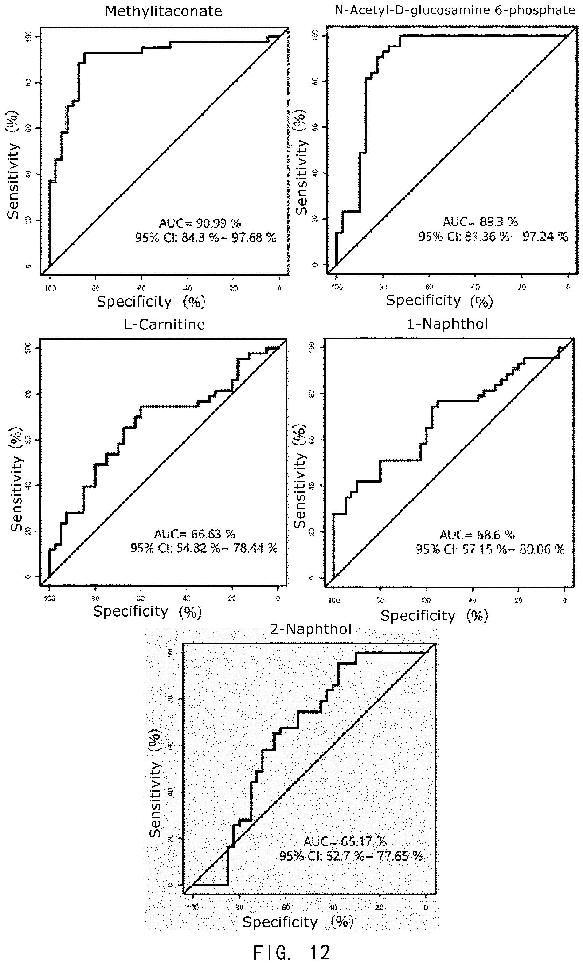

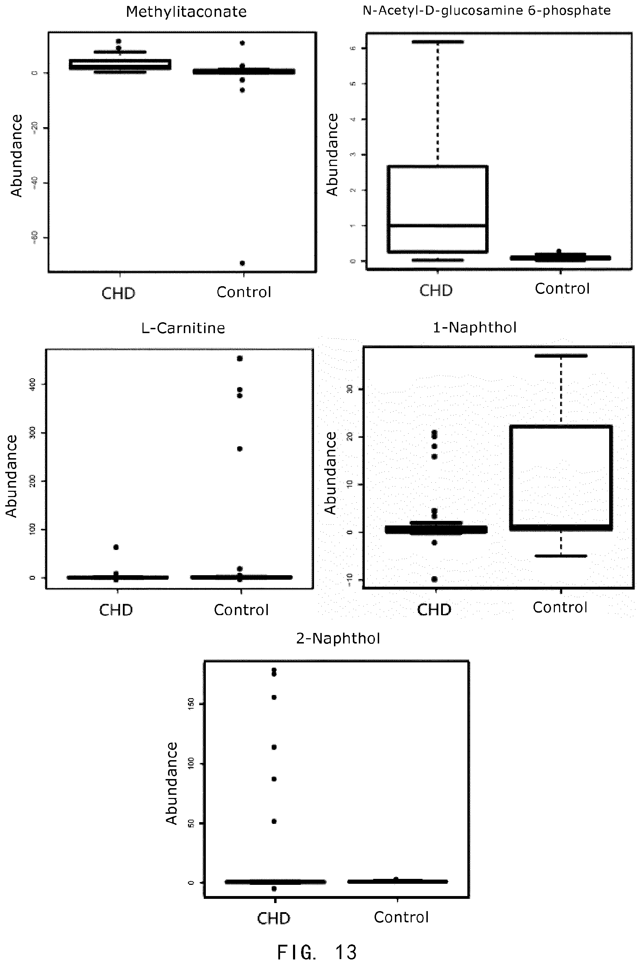

To evaluate the diagnostic capabilities of the seven potential biomarkers, the inventors analyzed another 102 plasma samples (validation set, 59 CHD patients and 43 healthy controls) using a receiver operating characteristic (ROC) analysis method. The inventor constructed a random forest classifier in the validation set, and found that among the seven potential biomarkers, combination of five potential biomarkers (methylitaconate, N-acetyl-D-glucosamine-6-phosphate, L-carnitine, 1-naphthol, and 2-Naphthol) have better discrimination capability, and the area under curve (AUC) thereof reached 89.95%, and 95% confidence interval (CI) was 83.29% -96.61% (Figure 10, Table 6). Table 5 has shown the abundance of the five metabolites in the CHD patient group and the control group of the validation set. As for the training set, the AUC of random forest classifiers for the five potential biomarkers was 97.44 with a 95% confidence interval (CI) of 94.69% -100% (Figure 11, Table 8). In the training set, the five metabolites were subjected to ROC analysis respectively. The results were shown in Fig. 12 and the AUCs were 90.99% (methylitaconate), 89.3% (N-acetyl-D-glucosamine -6-phosphate), 66.63% (L-carnitine), 68.6% (1-naphthol), and 65.17% (2-naphthol). Figure 13 has shown the abundance of the five metabolites in the CHD patient group and the control group of the training set (Table 7). The above results showed that the combination of the five metabolites as a discrimination capability of classifier is also very good for the training set.

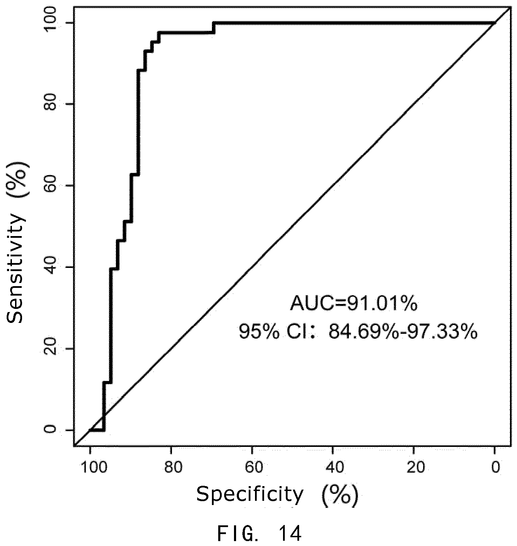

In addition, the inventors have found that both metabolites of N-acetyl-D-glucosamine-6-phosphate and 1-naphthol have greater potential as diagnostic markers for CHD. As a result, the inventors performed ROC analysis for the CHD combination diagnosis of the two metabolites in the validation set. The results (Figure 14) showed that the AUC reached 91.01% and the 95% confidence interval (CI) was 84.69% -97.33%, confirming that the combination of N-acetyl-D-glucosamine-6-phosphate and 1-naphthol has a higher ability to diagnose CHD

**Table 5 Ion intensity of the five metabolites in the Validation set samples**

| Number of samples (CD: CHD patients; N: healthy control) | 2-Naphthol | 1-Naphthol | L-Carnitine | Methylitaconate | N-Acetyl-D-glucosamine 6-phosphate |
|---|---|---|---|---|---|
| N1266_ 1_1 | 1.117101712 | 1.035859 | 0.676574 | 2.66610662 | 0.233469532 |
| CD6649_1_1 | 1.20018785 | 1.119556 | 1.088447 | 1.34596847 | 1.447698385 |
| CD6703_1_1 | 1.505156418 | 1.364263 | 1.22752 | 1.562735086 | 3.656078243 |
| CD6644_1_1 | 1.101113054 | 1.184177 | 0.853146 | 1.010583306 | 1.277471512 |
| CD6647_1_1 | 1.127316753 | 1.273934 | 1.184226 | 1.367266758 | 0.557392562 |
| N1262_ 1 | 1.494383535 | 1.62049 | 0.849435 | 2.383828056 | 0.147643317 |
| CD6650_1_1 | 1.032873232 | 1.098877 | 0.55428 | 1.451372669 | 0.327609289 |
| N1264_ 1_1 | 1.500069209 | 1.711231 | 0.800658 | 2.000754106 | 0.141283383 |
| N1296_ 1_1 | 1.270042546 | 1.28942 | 0.604219 | 1.20951021 | 0.201929255 |
| CD6598_1_1 | 1.21941452 | 1.383182 | 1.112506 | 2.126543886 | 1.3703207 |
| N1295_ 1_1 | 0.880308932 | 1.018959 | 1.080861 | 0.903309273 | 0.188292915 |
| N1263_ 1_1 | 1.344804364 | 1.435212 | 1.120406 | 1.757516083 | 0.228248005 |
| CD6584_1_1 | 0.86945552 | 0.851918 | 0.643016 | 0.51243288 | 0.260569751 |
| CD6586_1_1 | 0.89848935 | 0.999865 | 1.767907 | 0.912878165 | 2.059313131 |
| CD6733_1_1 | 1.146885421 | 1.204221 | 0.633313 | 1.207053891 | 0.216292814 |
| CD6601_1_1 | 0.900086382 | 1.002835 | 1.211885 | 1.126082621 | 1.868169829 |
| CD6636_1_1 | 0.930515297 | 1.118784 | 1.003791 | 1.034375536 | 0.171305691 |
| N1276_ 1_1 | 0.96505902 | 1.039842 | 1.145988 | 0.851925834 | 0.158936382 |
| N1261_ 1_1 | 1.031621685 | 1.078095 | 1.038145 | 1.032313387 | 0.158732489 |
| CD6700_1_1 | 0.847315706 | 1.092617 | 0.689922 | 0.756506575 | 0.808918648 |
| N1270_ 1_1 | 1.705761469 | 1.687215 | 0.948168 | 1.314462675 | 0.118940443 |
| CD8145_1_1 | 1.154624514 | 1.202943 | 1.063412 | 0.580430256 | 0.40742738 |
| N1267_ 1_1 | 1.491797746 | 1.474719 | 0.838126 | 1.953734078 | 0.130628359 |
| N1275_ 1_1 | 1.285295193 | 1.304741 | 1.039877 | 1.404869741 | 0.177367716 |
| CD6595_1_1 | 0.806751787 | 0.855489 | 1.066544 | 0.769515995 | 1.200823457 |
| N1307_ 1_1 | 1.157582362 | 1.152752 | 0.908811 | 0.539190632 | 0.054198375 |
| CD6645_1_1 | 1.099621172 | 1.031582 | 1.029575 | 1.139942549 | 1.830406868 |
| CD6657_1_1 | 0.709940326 | 0.823175 | 0.860845 | 0.714078589 | 0.251494406 |
| N1308_ 1_1 | 0.901973551 | 0.907191 | 1.037438 | 0.747140052 | 0.046508176 |
| CD6651_1_1 | 0.799434587 | 0.848028 | 1.015354 | 0.824739523 | 1.832639375 |
| N1269_ 1_1 | 0.942856213 | 0.970033 | 0.972331 | 0.934875264 | 0.119501265 |
| N1260_ 1_1 | 1.283601735 | 1.1993 | 1.077612 | 1.375475493 | 0.156782925 |
| N1268_ 1_1 | 1.200018193 | 1.227969 | 0.927194 | 1.304360059 | 0.108371558 |
| CD6643_1_1 | 0.580692626 | 0.567604 | 1.082437 | 1.069543923 | 4.840287283 |
| CD6722_1_1 | 1.151790636 | 1.076344 | 1.074231 | 0.920819669 | 0.999777686 |
| N1287_ 1_1 | 1.439882382 | 1.35139 | 1.084582 | 1.432884546 | 0.146257239 |
| N1277_ 1_1 | 2.227553873 | 1.992667 | 0.653869 | 1.859414874 | 0.200717511 |
| N1265_ 1_1 | 1.898536152 | 1.685059 | 0.501955 | 4.395443288 | 0.176639672 |
| CD6654_1_1 | 1.142916495 | 1.085113 | 1.411297 | 0.885056125 | 2.753568322 |
| CD6620_1_1 | 0.90951341 | 0.867033 | 1.448212 | 3.498473712 | 3.338767286 |
| N1285_ 1_1 | 1.227265818 | 1.209567 | 0.659973 | 1.99322123 | 0.165742571 |
| CD6582_2_2 | 0.945362227 | 0.878922 | 0.812159 | 0.567948349 | 0.477534628 |
| CD8321_2_2 | 1.796856016 | 1.240258 | 1.668886 | 1.654475052 | 0.113119379 |
| CD8185_2_2 | 1.152684977 | 0.998796 | 1.083636 | 1.060444513 | 1.974681065 |
| CD6591_2_2 | 1.312861304 | 1.244943 | 1.355744 | 1.558200434 | 1.357141074 |
| CD8380_2_2 | 1.193882342 | 1.20018 | 1.082078 | 2.44478792 | 4.494975017 |
| CD8118_2_2 | 1.518604114 | 1.410051 | 1.322469 | 1.426624876 | 2.411781784 |
| N1284_ 2_2 | 0.928755892 | 0.930666 | 1.220259 | 1.274893219 | 0.058981056 |
| N1293_2_2 | 1.2177694 | 1.269822 | 1.229655 | 2.139998607 | 0.038317117 |
| CD8331_2_2 | 1.035668941 | 1.18448 | 1.048285 | 2.023079151 | 0.507480306 |
| CD8243_2_2 | 0.599866749 | 0.720803 | 1.087798 | 1.084362454 | 1.554253283 |
| CD6610_2_2 | 1.157193385 | 1.29856 | 1.034842 | 2.174570013 | 3.255765104 |
| N1283_ 2_2 | 1.434227336 | 1.250797 | 1.130769 | 2.152953711 | 0.091090534 |
| N1309_ 2_2 | 1.169506318 | 1.097191 | 0.601285 | 4.150355032 | 0.029709158 |
| N1271_ 2_2 | 0.657228029 | 0.703753 | 0.850109 | 2.131344482 | 0.029009756 |
| CD8355_2_2 | 1.355281193 | 1.184011 | 0.899375 | 1.252550916 | 4.377711601 |
| N1272_ 2_2 | 1.292302712 | 0.898324 | 1.182961 | 1.062049142 | 0.061227639 |
| N1299_2_2 | 1.530310787 | 1.179142 | 1.071107 | 2.463762067 | 0.027782943 |
| CD8367_2_2 | 1.928389988 | 1.383243 | 1.116861 | 2.884627922 | 2.449459721 |
| N1291_ 2_2 | 1.198772826 | 1.013944 | 0.977248 | 1.035038195 | 0.023606458 |
| N1301_ 2_2 | 1.490192185 | 1.143835 | 0.95655 | 4.708562755 | 0.045495696 |
| N1280_ 2_2 | 0.790195259 | 0.855836 | 0.952208 | 1.140393408 | 0.052384013 |
| N1300_ 2_2 | 1.144893711 | 0.950703 | 0.9119 | 0.862827568 | 0.020540795 |
| N1297_2_2 | 1.460987556 | 0.963972 | 1.362675 | 1.319992162 | 0.025342354 |
| CD6716_2_2 | 1.481260035 | 1.001833 | 1.01364 | 1.371257837 | 1.047524432 |
| CD8117_2_2 | 1.166016087 | 0.910431 | 1.379624 | 1.1656302 | 1.845141131 |
| CD8264_2_2 | 1.382998308 | 1.010785 | 1.279019 | 0.693951317 | 0.284040149 |
| CD8119_2_2 | 1.141490485 | 0.890552 | 0.90961 | 0.753358868 | 0.046595478 |
| N1286_ 2_2 | 0.827329373 | 0.781236 | 0.837659 | 0.741240568 | 0.030016816 |
| CD8122_2_2 | 1.343837874 | 1.348491 | 1.275975 | 2.217999975 | 1.119939608 |
| CD8197_2_2 | 1.294409606 | 1.412058 | 1.339361 | 1.56422511 | 0.714297567 |
| CD8049_2_2 | 0.600520274 | 0.622965 | 1.152573 | 1.280262625 | 1.929193154 |
| CD8305_2_2 | 0.873897705 | 0.951066 | 0.915568 | 0.906708246 | 3.630904873 |
| N1282_ 2_2 | 1.466867165 | 1.406026 | 1.091289 | 3.102943122 | 0.00811708 |
| N1288_ 2_2 | 1.079520577 | 1.066332 | 1.315999 | 1.941844839 | 0.096093945 |
| CD6604_2_2 | 0.547231185 | 0.784313 | 1.111722 | 1.255790376 | 0.155151508 |
| CD6608_2_2 | 0.975888775 | 0.991372 | 0.712772 | 1.804251288 | 1.829255862 |
| CD8196_2_2 | 1.416250018 | 1.228571 | 0.853326 | 2.084370563 | 1.655784128 |
| CD8368_2_2 | 0.691122155 | 0.923728 | 0.634896 | 1.09664717 | 2.470573214 |
| CD8211_2_2 | 0.818078327 | 0.956718 | 1.050181 | 2.018976047 | 2.044872867 |
| CD8376_2_2 | 0.875553971 | 0.912671 | 1.082223 | 0.677934424 | 1.790676554 |
| CD8059_2_2 | 1.206772349 | 1.154504 | 1.242466 | 2.088586994 | 2.404911872 |
| N1298_ 2_2 | 0.956823496 | 0.898262 | 0.907729 | 1.030275584 | 0.069153719 |
| CD8239_2_2 | 0.782424486 | 1.099039 | 0.79366 | 0.679548656 | 0.507073895 |
| CD8144_2_2 | 0.997667975 | 1.095079 | 0.584135 | 0.286961675 | 0.909537224 |
| CD8350_2_2 | 1.022161728 | 1.187085 | 1.172763 | 3.793364455 | 3.42232517 |
| CD6594_2_2 | 0.546212422 | 0.599167 | 0.998506 | 0.75765484 | 2.366521709 |
| N1290_2_2 | 1.106542759 | 1.057568 | 1.258828 | 1.545863475 | 3.655578435 |
| N1279_2_2 | 1.173152953 | 1.051973 | 0.887975 | 0.563420277 | 0.017012144 |
| N1273_2_2 | 1.352979473 | 1.10814 | 1.278614 | 1.023851159 | 0.02863203 |
| CD8313_2_2 | 1.462510481 | 0.972833 | 1.069834 | 2.582851898 | 1.018119636 |
| CD8330_2_2 | 2.440802894 | 1.571521 | 1.060569 | 4.249812952 | 0.115896836 |
| CD8051_2_2 | 1.102376487 | 0.911256 | 0.91085 | 0.5960759 | 0.823533048 |
| CD8203_2_2 | 2.023329629 | 1.762102 | 1.53727 | 3.11455584 | 1.915010557 |
| CD8257_2_2 | 1.396910273 | 1.404206 | 1.08376 | 1.956493852 | 0.24599409 |
| N1294_ 2_2 | 1.41690455 | 1.379759 | 0.984985 | 3.264814173 | 0.023463843 |
| N1292_ 2_2 | 1.024975948 | 1.027951 | 1.083229 | 1.834964167 | 0.040089366 |
| N1274_ 2_2 | 1.310856602 | 1.243994 | 1.193563 | 2.532466726 | 0.057191588 |
| CD8149_2_2 | 0.894345876 | 0.966587 | 1.297199 | 1.6464641 | 0.018988019 |
| CD8212_2_2 | 0.899836844 | 0.746779 | 0.716142 | 0.609509399 | 1.038361585 |
| CD8234_2_2 | 0.953908965 | 0.869032 | 0.922416 | 1.185741978 | 0.470482996 |
| N1302_ 2_2 | 1.133956098 | 1.15785 | 1.524975 | 1.570394692 | 0.075152454 |

**Table 6 Prediction of the Probability of having diseases in Validation set by the combination of Five Metabolites (the subject is identified as the one who is in the risk of CHD or has CHD if the probability of having diseases ≥ 0.5)**

| Number of samples (CD: CHD patients; N: healthy control) | Probability of having diseases | Number of samples (CD: CHD patients; N: healthy control) | Probability of having diseases |
|---|---|---|---|
| N1266_ 1_1 | 0.614973 | CD6610_2_2 | 0.919355 |
| CD6649_1_1 | 0.934066 | N1283_ 2_2 | 0.06 |
| CD6703_1_1 | 0.79235 | N1309_ 2_2 | 0.221591 |
| CD6644_1_1 | 0.95082 | N1271_ 2_2 | 0.456731 |
| CD6647_1_1 | 0.891304 | CD8355_2_2 | 0.89375 |
| N1262_ 1_1 | 0.073864 | N1272_ 2_2 | 0.169492 |
| CD6650_1_1 | 0.846154 | N1299_2_2 | 0.062176 |
| N1264_ 1_1 | 0.036649 | CD8367_2_2 | 0.90625 |
| N1296_ 1_1 | 0.347594 | N1291_ 2_2 | 0.086207 |
| CD6598_1_1 | 0.931818 | N1301_ 2_2 | 0.171271 |
| N1295_ 1_1 | 0.632768 | N1280_ 2_2 | 0.485876 |
| N1263_ 1_1 | 0.541436 | N1300_ 2_2 | 0.340541 |
| CD6584_1_1 | 0.892857 | N1297_2_2 | 0.284946 |
| CD6586_1_1 | 0.910828 | CD6716_2_2 | 0.913265 |
| CD6733_1_1 | 0.137255 | CD8117_2_2 | 0.918782 |
| CD6601_1_1 | 0.95082 | CD8264_2_2 | 0.720812 |
| CD6636_1_1 | 0.080402 | CD8119_2_2 | 0.011236 |
| N1276_ 1_1 | 0.217647 | N1286_ 2_2 | 0.414773 |
| N1261_ 1_1 | 0.305263 | CD8122_2_2 | 0.895604 |
| CD6700_1_1 | 0.964646 | CD8197_2_2 | 0.900585 |
| N1270_ 1_1 | 0.253012 | CD8049_2_2 | 0.945652 |
| CD8145_1_1 | 0.977654 | CD8305_2_2 | 0.838323 |
| N1267_ 1_1 | 0.047619 | N1282_ 2_2 | 0.172222 |
| N1275_ 1_1 | 0.159341 | N1288_ 2_2 | 0.276596 |
| CD6595_1_1 | 0.958084 | CD6604_2_2 | 0.244898 |
| N1307_ 1_1 | 0.34104 | CD6608_2_2 | 0.91623 |
| CD6645_1_1 | 0.845745 | CD8196_2_2 | 0.845745 |
| CD6657_1_1 | 0.851429 | CD8368_2_2 | 0.953846 |
| N1308_ 1_1 | 0.448276 | CD8211_2_2 | 0.952096 |
| CD6651_1_1 | 0.978947 | CD8376_2_2 | 0.971591 |
| N1269_ 1_1_ | 0.288889 | CD8059_2_2 | 0.928571 |
| N1260_ 1_1 | 0.116022 | N1298_ 2_2 | 0.265403 |
| N1268_ 1_1 | 0.105 | CD8239_2_2 | 0.963542 |
| CD6643_1_1 | 0.847716 | CD8144_2_2 | 0.945946 |
| CD6722_1_1 | 0.916667 | CD8350_2_2 | 0.911602 |
| N1287_ 1_1 | 0.140244 | CD6594_2_2 | 0.949239 |
| N1277_ 1_1 | 0.361446 | N1290_2_2 | 0.914894 |
| N1265_ 1_1 | 0.283654 | N1279_2_2 | 0.221591 |
| CD6654_1_1 | 0.913514 | N1273_2_2 | 0.1875 |
| CD6620_1_1 | 0.838889 | CD8313_2_2 | 0.902299 |
| N1285_ 1_1 | 0.132948 | CD8330_2_2 | 0.08427 |
| CD6582_2_2 | 0.954023 | CD8051_2_2 | 0.943299 |
| CD8321_2_2 | 0.16185 | CD8203_2_2 | 0.815789 |
| CD8185_2_2 | 0.947368 | CD8257_2_2 | 0.385787 |
| CD6591_2_2 | 0.93299 | N1294_ 2_2 | 0.043716 |
| CD8380_2_2 | 0.881443 | N1292_ 2_2 | 0.119565 |
| CD8118_2_2 | 0.88764 | N1274_ 2_2 | 0.055556 |
| N1284_ 2_2 | 0.315217 | CD8149_2_2 | 0.156069 |
| N1293_2_2 | 0.082418 | CD8212_2_2 | 0.967213 |
| CD8331_2_2 | 0.920213 | CD8234_2_2 | 0.882022 |
| CD8243_2_2 | 0.978261 | N1302_ 2_2 | 0.52459 |

**Table 7 Ion intensity of the five metabolites in the training set samples**

| Number of samples (CD: CHD patients; N: healthy control) | 1-Naphthol | 2-Naphthol | L-Carnitine | Methylitaconate | N-Acetyl-D-glucosamine 6-phosphate |
|---|---|---|---|---|---|
| CD5751_1 | 1.919931575 | -0.126581486 | 1.055084949 | 7.675836154 | 0.154406054 |
| CD5767_1 | 0.109204987 | -4.723527828 | 0.55634743 | 9.063103549 | 1.078396238 |
| CD5778_1 | 0.278703446 | -0.017620717 | 0.839535135 | 11.54731525 | 0.428768509 |
| CD5779_1 | -9.816962302 | -0.011837539 | 0.708983577 | 9.107544696 | 1.493180395 |
| CD5782_1 | -0.305192714 | -0.01444448 | 0.58355112 | 7.013951028 | 3.650638666 |
| CD5783_1 | -0.155896633 | -0.014991272 | 0.913003572 | 11.60286613 | 0.194757753 |
| CD5788_1 | 0.152840798 | 0.155038963 | -1.394621698 | 3.633374252 | 0.522010833 |
| CD5795_1 | 0.677722406 | 0.00424882 | -4.178798283 | 4.47855234 | 6.180111038 |
| CD5796_1 | 0.030469982 | 0.00436572 | 8.489291225 | 4.511846041 | 0.614399825 |
| CD5797_1 | 0.591813271 | 1.448798499 | 1.327866744 | 3.893171902 | 1.401345783 |
| CD5805_1 | 0.913556022 | 0.032021985 | 1.399700741 | 2.247166558 | 1.174037154 |
| CD5814_1 | 0.069094842 | 51.68728361 | 0.340906928 | 2.901569565 | 0.923042863 |
| CD5816_1 | 0.033245683 | 0.788603689 | 0.001067405 | 2.977008687 | 1.661642418 |
| CD5819_1 | 0.522030222 | 114.0020237 | 0.434029732 | 2.959772208 | 0.04640559 |
| CD5822_1 | 0.735187089 | 155.726217 | 0.945621373 | 2.196660559 | 1.788736731 |
| CD5831_1 | 1.014437469 | 0.793861845 | 0.688816058 | 2.373092556 | 3.433064628 |
| CD5832_1 | 0.039907611 | 0.710935343 | 0.868602077 | 1.815940085 | 0.269311421 |
| CD5833_1 | 1.158389151 | 0.587940137 | 0.93440022 | 0.377771024 | 3.737536286 |
| CD5838_1 | 0.084543634 | 0.590185165 | 1.040777937 | 2.467266574 | 0.783109069 |
| CD5851_1 | 3.372372684 | 0.798962463 | 0.773800475 | 2.341071211 | 2.241677054 |
| CD5860_1 | 4.472314376 | 87.14539315 | 0.001374821 | 2.402902841 | 4.624038101 |
| CD5863_1 | 0.545525378 | 1.025263612 | 0.756479592 | 1.715297072 | 0.025052178 |
| CD5867_1 | 1.015839255 | 1.244424544 | 0.896337893 | 1.676303741 | 0.343626948 |
| CD5871_1 | 18.0374075 | 175.2432428 | 0.738027271 | 1.220907844 | 3.158325066 |
| N212E_ 1 | 36.95648286 | 1.559070443 | 0.002012133 | 0.362269489 | 0.022841137 |
| N213E_ 1 | 0.820412074 | 1.337154802 | 0.856819467 | 0.90701106 | 0.281422765 |
| N214E_ 1 | 0.904069739 | 1.17718265 | 1.006455525 | 0.661770359 | 0.068412451 |
| N215E_ 1 | 19.73266574 | 0.818298924 | 0.865818748 | 0.135950012 | 0.188238362 |
| N217E_ 1 | 30.38236617 | 1.54405141 | 1.253070817 | 0.833328673 | 0.051701596 |
| N218E_ 1 | 25.39095508 | 1.275781279 | 1.172011127 | 0.154069919 | 0.114508699 |
| N220E_ 1 | 1.188703132 | 2.220195187 | 0.964618292 | 1.058164373 | 0.089354399 |
| N222E_1 | 0.712126632 | 1.368318052 | 0.918479305 | 0.678444592 | 0.171668456 |
| N223E_ 1 | 25.66238168 | 1.048358012 | 0.001421325 | 0.174312817 | 0.108321251 |
| N226E_ 1 | 0.714341495 | 1.551713961 | 0.59290109 | 0.310019992 | 0.024322491 |
| N227E_ 1 | 23.3354304 | 1.104457016 | 0.826290711 | 0.132174284 | 0.020484993 |
| N228E_ 1 | 19.06943068 | 0.832275171 | 0.93342376 | 0.947107227 | 0.088105391 |
| N229E_ 1 | 22.55353322 | 0.97442588 | 0.951781973 | 0.475540309 | 0.018666081 |
| N231E_ 1 | 0.762961197 | 0.912743199 | 1.170366222 | 0.507135006 | 0.207481925 |
| N232E_ 1 | 25.34816236 | 1.226135057 | 1.263796385 | 0.543345896 | 0.105066556 |
| N233E_ 1 | 21.71538323 | 0.981785788 | 3.974980561 | 0.17224438 | 0.040789811 |
| N234E_ 1 | 0.847298451 | 1.190218138 | 0.013119811 | 0.68788123 | 0.068448621 |
| N235E_ 1 | 27.35957282 | 1.33169256 | 389.159741 | 0.096476072 | 0.105870133 |
| N236E_ 1 | 19.64610247 | 0.617901586 | 267.1120223 | 0.387318025 | 0.052856433 |
| N237E_1 | 0.902429584 | 1.026136287 | 454.722355 | 0.993155163 | 0.069773832 |
| N238E_ 1 | 0.716095906 | 0.993307269 | 376.5739741 | 0.103040963 | 0.015568699 |
| N239E_ 1 | 24.36629963 | 0.755874187 | 453.0168626 | 1.091751207 | 0.092074916 |
| N241E_ 1 | 0.17938809 | 0.914495326 | 18.51087103 | 1.090353717 | 0.089658868 |
| N242E_ 1 | 0.069647359 | 1.090274222 | 4.286614113 | 0.996506292 | 0.193374012 |
| N243E_ 1 | 0.045143326 | 2.105294114 | 0.004943757 | 0.131086742 | 0.074958436 |
| N244E_ 1 | 0.030498972 | 3.132531095 | 1.557186979 | 0.083930243 | 0.131496597 |
| N245E_ 1 | 0.698950825 | 1.88873827 | 0.966610095 | 0.065982364 | 0.274324854 |
| CD5877_2 | -0.16732147 | 1.241464791 | 0.0039141 | 7.164066107 | 2.674426156 |
| CD5881_2 | -0.072330328 | 0.866316876 | 2.33705738 | 9.043076379 | 3.594712894 |
| CD5884_2 | -0.155685467 | 1.28608629 | 63.00039997 | 2.763528257 | 0.671412692 |
| CD5891_2 | -0.239757746 | 0.746038236 | -3.943373921 | 1.427877184 | 0.247741343 |
| CD5892_2 | -2.182340094 | 0.911877693 | -2.673659246 | 6.328931707 | 0.128295792 |
| CD5898_2 | 0.454948579 | 0.956623781 | 0.6600458 | 3.737508016 | 0.048169769 |
| CD5900_2 | 20.00785145 | 0.866613265 | 0.777262201 | 2.394818234 | 2.65812009 |
| CD5916_2 | 0.528395737 | 0.520492404 | 0.392023852 | 3.221713678 | 0.608166193 |
| CD5923_2 | 0.82554758 | 0.969902323 | 0.834946584 | 1.364474659 | 1.8076432 |
| CD5925_2 | 0.428843518 | 0.416137722 | 0.811408313 | 1.709302548 | 0.086980503 |
| CD5926_2 | 15.85107425 | 178.8427667 | 0.6430885 | 1.700470622 | 0.041627397 |
| CD5931_2 | 20.87193369 | 1.036447476 | 0.751287814 | 1.789807723 | 3.565204502 |
| CD5934_2 | 1.147972107 | 0.79537622 | 1.332254396 | 0.937139278 | 4.240267701 |
| CD5935_2 | 1.980852593 | 0.673314704 | 0.587142794 | 0.537742629 | 1.687371607 |
| CD5988_2 | 1.145020029 | 0.587776213 | 0.684805037 | 0.904939973 | 0.20841871 |
| CD5990_2 | 0.921324749 | 0.835092372 | -2.925977143 | 0.680035894 | 0.844215022 |
| N165E_ 2 | 12.54402826 | 0.626124671 | -3.939716918 | 0.443320649 | 0.073093648 |
| N167E_2 | 0.609538409 | 0.895469187 | 5.072059626 | 0.546320264 | 0.100961447 |
| N168E_ 2 | 17.99656969 | 0.8077213 | 1.662580458 | 0.560952486 | 0.058834087 |
| N170E_2 | 13.08883047 | 0.569678358 | 1.231604258 | 0.077404925 | 0.037618694 |
| N171E_ 2 | 0.736108561 | 0.60866081 | 0.001679314 | -69.27402018 | 0.075662903 |
| N185E_ 2 | 1.50439584 | 1.164581197 | 0.804110609 | 11.00289168 | 0.05885842 |
| N186E_ 2 | 28.91528304 | 0.640340132 | 0.532893614 | 2.614966334 | 0.041267076 |
| N187E_ 2 | 35.9280246 | 0.5238016 | 0.564265578 | 1.263532907 | 0.227687856 |
| N190E_2 | 1.465205631 | 0.684486204 | 0.877206282 | 1.01653419 | 0.112743104 |
| N191E_ 2 | 0.118765283 | 0.73855797 | 0.87355162 | 0.775146553 | 0.111178163 |
| N195E_ 2 | 1.837239183 | 0.759006694 | 0.001726182 | 0.852832069 | 0.023017773 |
| N197E_2 | 0.034343229 | 0.934984316 | 5.250385231 | 0.719662968 | 0.027642139 |
| N200E_ 2 | -0.123405532 | 1.439600164 | 0.841363907 | -6.189816789 | 0.099738245 |
| N207E_ 2 | -0.06709231 | 1.022930088 | 0.000675396 | -2.529032677 | 0.090746589 |
| N247E_2 | -4.940484379 | 0.714652667 | 0.834643138 | 2.31543238 | 0.113118963 |
| N248E_ 2 | -2.841907603 | 1.029665429 | 0.796745767 | 1.268977536 | 0.065344673 |

**Table 8 Prediction of the Probability of having diseases in Training Set by the combination of Five Metabolites (the subject is identified as the one who is in the risk of CHD or has CHD if the probability of having diseases ≥ 0.5)**

| Number of samples (CD: CHD patients; N: healthy control) | Probability of having diseases | Number of samples (CD: CHD patients; N: healthy control) | Probability of having diseases |
|---|---|---|---|
| CD5751_1 | 0.606741573 | N236E_ 1 | 0.132352941 |
| CD5767_1 | 0.995073892 | N237E_ 1 | 0.0375 |
| CD5778_1 | 1 | N238E_ 1 | 0.030612245 |
| CD5779_1 | 0.931578947 | N239E_ 1 | 0.036809816 |
| CD5782_1 | 1 | N241E_ 1 | 0.040229885 |
| CD5783_1 | 0.864583333 | N242E_ 1 | 0.216346154 |
| CD5788_1 | 0.994382022 | N243E_ 1 | 0.08045977 |
| CD5795_1 | 0.982857143 | N244E_ 1 | 0.033898305 |
| CD5796_1 | 0.988095238 | N245E_ 1 | 0.280701754 |
| CD5797_1 | 0.862944162 | CD5877_2 | 0.940540541 |
| CD5805_1 | 0.941520468 | CD5881_2 | 0.988826816 |
| CD5814_1 | 0.97382199 | CD5884_2 | 0.838150289 |
| CD5816_1 | 0.82967033 | CD5891_2 | 0.71657754 |
| CD5819_1 | 0.793478261 | CD5892_2 | 0.653846154 |
| CD5822_1 | 0.878787879 | CD5898_2 | 0.772727273 |
| CD5831_1 | 0.994708995 | CD5900_2 | 0.907514451 |
| CD5832_1 | 0.772020725 | CD5916_2 | 0.979166667 |
| CD5833_1 | 0.777173913 | CD5923_2 | 0.984042553 |
| CD5838_1 | 0.954314721 | CD5925_2 | 0.368421053 |
| CD5851_1 | 0.87431694 | CD5926_2 | 0.244897959 |
| CD5860_1 | 0.723958333 | CD5931_2 | 0.861878453 |
| CD5863_1 | 0.232044199 | CD5934_2 | 0.755952381 |
| CD5867_1 | 0.704918033 | CD5935_2 | 0.803191489 |
| CD5871_1 | 0.775147929 | CD5988_2 | 0.256281407 |
| N212E_ 1 | 0.141414141 | CD5990_2 | 0.703488372 |
| N213E_ 1 | 0.395721925 | N165E_ 2 | 0.218390805 |
| N214E_ 1 | 0.136904762 | N167E_2 | 0.162921348 |
| N215E_ 1 | 0.214285714 | N168E_ 2 | 0.152542373 |
| N217E_ 1 | 0.011363636 | N170E_2 | 0.437810945 |
| N218E_1 | 0.010471204 | N171E_ 2 | 0.519774011 |
| N220E_ 1 | 0.037267081 | N185E_ 2 | 0.109375 |
| N222E_ 1 | 0.029761905 | N186E_ 2 | 0.344086022 |
| N223E_ 1 | 0.066298343 | N187E_ 2 | 0.577319588 |
| N226E_ 1 | 0.146596859 | N190E_2 | 0.263157895 |
| N227E_ 1 | 0.126436782 | N191E_ 2 | 0.086705202 |
| N228E_ 1 | 0.034482759 | N195E_ 2 | 0.143678161 |
| N229E_ 1 | 0.028901734 | N197E_2 | 0.163934426 |
| N231E_ 1 | 0.209677419 | N200E_ 2 | 0.112903226 |
| N232E_ 1 | 0 | N207E_ 2 | 0.164102564 |
| N233E_ 1 | 0.016666667 | N247E_ 2 | 0.505617978 |
| N234E_ 1 | 0.170984456 | N248E_ 2 | 0.132596685 |
| N235E_ 1 | 0.005617978 | | |

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A set of biomarkers comprising one or more biomarker(s) selected from the list consisting of:
| **Number** | **Name** | **Molecular Formula** | **m/z** | **retention time RT (s)** |
|---|---|---|---|---|
| Biomarkerb1 | 1-Naphthol | C₁₀H₈O | 145.0649552 | 531.467 |
| Biomarker b2 | 2-Naphthol | C₁₀H₈O | 144.0571958 | 531.9175 |
| Biomarker b3 | L-Carnitine | C₇H₁₅NO₃ | 162.1122492 | 114.494 |
| Biomarker b4 | Methylitaconate | C₆H₈O₄ | 167.0315121 | 104.76 |
| Biomarker b5 | N-Acetyl-D-glucosamine 6-phosphate | C₈H₁₆NO₉P | 324.0458651 | 541.3545 |
| Biomarker b6 | 2,3-Dimethylmaleate | C₆H₈O₄ | 145.0496085 | 1156.85 |
| Biomarker b7 | Phenylpyruvate | C₉H₈O₃ | 165.0548439 | 183.8775 |
| Biomarker b8 | 20,26-Dihydroxyecdysone | C₂₇H₄₄O₈ | 496.3058791 | 744.104 |
| Biomarker b9 | Cortol | C₂₁H₃₆O₅ | 386.2897576 | 662.962 |
| Biomarker b10 | 10-Hydroxydihydrosanguinarine | C₂₀H₁₅NO₅ | 372.0845309 | 684.837 |
| Biomarker b11 | Progesterone | C₂₁H₃₀O₂ | 337.215992 | 774.709 |
| Biomarker b12 | (9Z,11E)-(13S)-13-Hydroperoxyoctadeca-9,11- dienoic acid | C₁₈H₃₂O₄ | 330.2635353 | 653.076 |
| Biomarker b13 | Phytosphingosine | C₁₈H₃₉NO₃ | 318.2999289 | 656.764 |
| Biomarker b14 | p-Coumaroylagmatine | C₁₄H₂₀N₄O₂ | 315.1211881 | 145.31 |
| Biomarker b15 | N6-(L-1,3-Dicarboxypropyl)-L-lysine | C₁₁H₂₀N₂O₆ | 315.096019 | 142.1965 |
| Biomarker b16 | Dihydrokaempferol | C₁₅H₁₂O₆ | 311.0511119 | 611.26 |
| Biomarker b17 | Indoleglycerol phosphate | C₁₁H₁₄NO₆P | 310.0474631 | 611.2495 |
| Biomarker b18 | Medicarpin | C₁₆H₁₄O₄ | 309.0542143 | 611.434 |
| Biomarker b19 | 2-Carboxy-2-hydroxy-8-carboxychromene | C₁₁H₈O₆ | 274.9929078 | 91.3841 |
| Biomarker b20 | Hexadecanoic acid | C₁₆H₃₂O₂ | 274.2736949 | 656.679 |
| Biomarker b21 | Xanthoxin | C₁₅H₂₂O₃ | 268.1909849 | 571.68 |
| Biomarker b22 | 5-(3'-Carboxy-3'-oxopropyl)-4,6-dihydroxypicolinate | C₁₀H₉NO₇ | 255.0378919 | 117.92 |
| Biomarker b23 | N-Acetyl-L-2-amino-6-oxopimelate | C₉H₁₃NO₆ | 249.1082592 | 117.643 |
| Biomarker b24 | 4-(L-Alanin-3-yl)-2-hydroxy-cis, cis-muconate 6-semialdehyde | C₉H₁₁NO₆ | 247.0927331 | 146.379 |
| Biomarker b25 | 4-[(Hydroxymethyl)nitrosoamino]-1-(3-pyridinyl)-1-butanone | C₁₀H₁₃N₃O₃ | 223.0935157 | 644.408 |
| Biomarker b26 | Ecgonine | C₉H₁₅NO₃ | 203.1391048 | 119.338 |
| Biomarker b27 | Creatine | C₄H₉N₃O₂ | 170.0328456 | 202.182 |
| Biomarker b28 | 7-Methylxanthine | C₆H₆N₄O₂ | 167.0564801 | 502.8765 |
| Biomarker b29 | Cyromazine | C₆H₁₀N₆ | 166.0956757 | 485.317 |
| Biomarker b30 | 3-Fluorocatechol | C₆FH₅O₂ | 146.0600839 | 531.627 |
| Biomarker b31 | Thymine | C₅H₆N₂O₂ | 144.0761483 | 532.091 |
| Biomarker b32 | Sulfoacetaldehyde | C₂H₄O₄S | 123.9826417 | 769.939 |
| Biomarker b33 | 2-Oxobutanoate | C₄H₆O₃ | 103.0389661 | 114.685 |
| Biomarker b34 | N-Formiminoglycine | C₃H₆N₂O₂ | 103.0500104 | 485.3545 |
| Biomarker b35 | 2,4-Diene-VPA | C₈H₁₂O₂ | 141.0908185 | 634.193 |
| Biomarker b36 | 3-Dimethylallyl-4- hydroxybenzoate | C₁₂H₁₄O₃ | 206.0948611 | 531.681 |
| Biomarker b37 | Aphidicolin | C₂₀H₃₄O₄ | 356.2792855 | 666.068. |

2. The set of claim 1, wherein the set comprises biomarker bm and one or more biomarker(s) selected from a subset X, wherein the subset X consists of biomarkers b1 to b(m-1) and biomarkers b(m+1) to b37, m is an integer and 1≤m≤37, preferably 1≤m≤7, and more preferably 1≤m≤5.

3. The set of claim 1, wherein the set comprises biomarker(s) selected from the group consisting of:
biomarker b1, biomarker b2, biomarker b3, biomarker b4, biomarker b5, biomarker b6, biomarker b7, and the combination thereof.

4. The set of claim 1, wherein the set comprises biomarker(s) selected from the group consisting of:
biomarker b1, biomarker b2, biomarker b3, biomarker b4, biomarker b5, and the combination thereof.

5. The set of claim 1, wherein compared with a reference value, one or more biomarker(s) selected from a subset H is (are) increased indicating that the test subject is in the risk of CHD or has CHD,
wherein the subset H includes biomarkers b2, b4, b5, b6, b8, b10, b15, b16, b17, b18, b21, b22, b23, b24, b26, b27, b31, b32, b33, b34, and b35.

6. The set of claim 1, wherein compared with a reference value, one or more biomarker(s) selected from a subset H is (are) decreased, indicating that the test subject is in the risk of CHD or has CHD,
wherein the subset K includes biomarkers b1, b3, b7, b9, b11, b12, b13, b14, b19, b20, b25, b28, b29, b30, b36 and b37.

7. The set of claim 1, wherein the set is used for the evaluation on the risk of CHD or CHD diagnosis in a test subject.

8. A reagent combination for evaluating or diagnosing a risk of CHD, wherein the reagent combination comprises a reagent for detecting each biomarker in the set according to claim 1.

9. A kit comprising the set according to claim 1 and/or the reagent combination according to claim 8.

10. Use of the set according to claim 1 and/or the reagent combination according to claim 8 for preparing a kit, wherein the kit is used for the evaluation on a risk of CHD or CHD diagnosis in a test subject.

11. The use of claim 10, wherein the evaluation and diagnosis further comprises the following steps:
(1) providing a sample from a test subject and detecting the level of each biomarker in the set according to claim 1 in the sample;
(2) comparing the level detected in step (1) with a reference dataset or a reference value (such as a reference value of a healthy control);
preferably, the reference dataset includes the levels of each biomarker in the set according to claim 1 from CHD patients and healthy controls.

12. The use of claim 10, wherein the sample is selected from the group consisting of: blood, plasma, and serum.

13. A method for evaluating a risk of CHD or diagnosing CHD in a test subject, comprising the steps of:
(1) providing a sample from a test subject and detecting the level of each biomarker in the set according to claim 1 in the sample;
(2) comparing the level detected in step (1) with a reference dataset or a reference value (such as a reference value of a healthy control);
preferably, the reference dataset includes the levels of each biomarker in the set according to claim 1 from CHD patients and healthy controls.

14. A method for screening a candidate compound for the treatment of CHD, comprising the steps of:
(1) in a test group, administering a test compound to a test subject, and detecting the level VI of each biomarker in the set according to claim 1 from the sample of the subject in the test group; and in a control group, administering a blank control (including a vehicle) to a test subject, and detecting the level V2 of each biomarker in the set according to claim 1 from the sample of the subject in the control group;
(2) comparing the level VI with the level V2 detected in the previous step to determine whether the test compound is a candidate compound for the treatment of CHD
